(19) Europäisches Patentamt
European Patent Office
Office européen des brevets

(11) **EP 2 829 613 A1**

(12) **EUROPEAN PATENT APPLICATION**

(43) Date of publication:
**28.01.2015 Bulletin 2015/05**

(51) Int Cl.:
**C12Q 1/68** (2006.01)

(21) Application number: **13178245.0**

(22) Date of filing: **26.07.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Istituto Nazionale Di Genetica Molecolare-INGM**
**20122 Milano (IT)**

(72) Inventors:
• **Abrignani, Sergio**
**53040 Serre di Rapolano (SI) (IT)**

• **Pagani, Massimiliano**
**24069 Trescore Balneario (BG) (IT)**
• **De Francesco, Raffaele**
**20146 Milano (IT)**

(74) Representative: **Capasso, Olga et al**
**De Simone & Partners S.p.A.**
**Via Vincenzo Bellini, 20**
**00198 Roma (IT)**

Remarks:
If details are not fully discernible, that is due to the quality of the drawingsfiled. The meaningfulness of the drawings is the applicant's responsibility.

(54) **Biomarkers of liver diseases and uses thereof**

(57) The present invention relates to miR-122 and miR-885-5p and their use in diagnostic and/or prognostic methods of liver diseases as well as modulators of miR-885-Sp for the treatment and/or prevention of a liver disease.

FIG. 6

**EP 2 829 613 A1**

SEQUENCE LISTING

<110>  ISTITUTO NAZIONALE DI GENETICA MOLECOLARE – INGM

<120>  Biomarkers of liver diseases and uses thereof

<130>  BE 120826

<160>  25

<170>  PatentIn version 3.5

<210>  1
<211>  22
<212>  RNA
<213>  Homo sapiens

<400>  1
uggaguguga caaugguguu ug                                                    22


<210>  2
<211>  22
<212>  RNA
<213>  Homo sapiens

<400>  2
uauagggauu ggagccgugg cg                                                    22


<210>  3
<211>  22
<212>  RNA
<213>  Homo sapiens

<400>  3
gcuacuucac aacaccaggg cc                                                    22


<210>  4
<211>  22
<212>  RNA
<213>  Homo sapiens

<400>  4
cagugguuuu acccuauggu ag                                                    22


<210>  5
<211>  22
<212>  RNA
<213>  Homo sapiens

<400>  5
uagcagcacg uaaauauugg cg                                                    22


<210>  6
<211>  21
<212>  RNA
<213>  Homo sapiens

<400>  6

```
caucccuugc augguggagg g                                           21


<210>   7
<211>   21
<212>   RNA
<213>   Homo sapiens


<400>   7
uagcagcaca gaaauauugg c                                           21


<210>   8
<211>   22
<212>   RNA
<213>   Homo sapiens


<400>   8
uucaccaccu ucuccaccca gc                                          22


<210>   9
<211>   23
<212>   RNA
<213>   Homo sapiens


<400>   9
ugugcaaauc uaugcaaaac uga                                         23


<210>   10
<211>   23
<212>   RNA
<213>   Homo sapiens


<400>   10
ugugcaaauc caugcaaaac uga                                         23


<210>   11
<211>   23
<212>   RNA
<213>   Homo sapiens


<400>   11
aguuuugcag guuugcaucc agc                                         23


<210>   12
<211>   22
<212>   RNA
<213>   Homo sapiens


<400>   12
uagcuuauca gacugauguu ga                                          22


<210>   13
<211>   22
<212>   RNA
<213>   Homo sapiens


<400>   13
```

ugucaguuug ucaaauaccc ca                                                  22


<210>    14
<211>    22
<212>    RNA
<213>    Homo sapiens


<400>    14
uggcucaguu cagcaggaac ag                                                  22


<210>    15
<211>    23
<212>    RNA
<213>    Homo sapiens


<400>    15
uguaaacauc cuacacucuc agc                                                 23


<210>    16
<211>    21
<212>    RNA
<213>    Homo sapiens


<400>    16
cacauuacac ggucgaccuc u                                                   21


<210>    17
<211>    23
<212>    RNA
<213>    Homo sapiens


<400>    17
ucaagagcaa uaacgaaaaa ugu                                                 23


<210>    18
<211>    21
<212>    RNA
<213>    Homo sapiens


<400>    18
acuggacuug gagucagaag g                                                   21


<210>    19
<211>    22
<212>    RNA
<213>    Homo sapiens


<400>    19
aagacgggag gaaagaaggg ag                                                  22


<210>    20
<211>    22
<212>    RNA
<213>    Homo sapiens


<400>    20

ugauugguac gucugugggu ag                                                          22

```
<210>   21
<211>   21
<212>   RNA
<213>   Homo sapiens

<400>   21
```
ugaguuggcc aucugaguga g                                                           21

```
<210>   22
<211>   21
<212>   RNA
<213>   Homo sapiens

<400>   22
```
ugagcuaaau gugugcuggg a                                                           21

```
<210>   23
<211>   19
<212>   RNA
<213>   Homo sapiens

<400>   23
```
ucuaggcugg uacugcuga                                                              19

```
<210>   24
<211>   20
<212>   RNA
<213>   Homo sapiens

<400>   24
```
cggcucuggg ucugugggga                                                             20

```
<210>   25
<211>   22
<212>   RNA
<213>   Homo sapiens

<400>   25
```
uccauuacac uacccugccu cu                                                          22

## Description

**Technical field**

The present invention relates to miR-122 and miR-885-5p and their use in diagnostic and/or prognostic methods of liver diseases as well as modulators of miR-885-5p for the treatment and/or prevention of a liver disease.

**Background art**

MicroRNAs (miRNAs) are small noncoding RNAs of 22-25 nucleotides that regulate gene expression by binding mostly the 3' untranslated region (3'UTR) of target mRNAs. They are broadly conserved and act post-transcriptionally directing target gene repression [1,2]. Their impaired expression has been associated with a variety of diseases, including cancers, inflammation and chronic viral infections [3-5]. Moreover, in some tissues particular miRNAs are expressed in specific manner, and correlate to disease condition [6,7,3]. miRNAs have also been stably detected in the serum or plasma of healthy donors and patients and their levels are poorly influenced by multiple freeze-thaw cycles, long term storage or treatment with RNase before extraction [8-10]. Accumulating evidences support the use of miRNAs circulating in blood as non-invasive biomarkers of disease conditions, particularly cancer [11-13]. Moreover, in some tissues particular miRNAs are expressed in specific manner, correlating to disease condition [6,7,3]. hence as a promising alternative for the diagnosis of several pathological conditions and the monitoring of disease progression.

Hepatitis C Virus (HCV) is a positive-strand RNA virus that establishes a persistent infection in more than 70% of people who contract it. The chronic infection progresses to severe liver disease in about 20% of the persistently infected subjects, with development of liver cirrhosis and hepatocellular carcinoma (HCC). HCC is the leading cause of death among cirrhotic patients and the third cause of cancer-related mortality [14]. Standard therapy with Pegylated Interferon-y and ribavirin fails to clear HCV in $\sim 50\%$ of chronically infected individuals [15,16]. Moreover, no vaccination is currently available. Classical biochemical markers to follow disease progression show limited potential mainly due to their invasiveness or little specificity and sensitivity, especially for HCC. Alanine aminotransferase (ALT) activity in the blood is the most widely used biochemical marker for measuring liver damage. However, ALT levels may fluctuate during HCV infection and values may even fall back into normal range [17]. Moreover, ALT has been shown to be increased in clinical disorders other than liver diseases [18,19]. $\alpha$-Fetoprotein ($\alpha$-AFP) has long been used in clinic for the diagnosis of primary HCC, showing nonetheless unsatisfying sensitivity and specificity [20]. Furthermore, elevated AFP levels may be observed in patients with cirrhosis or flares of active hepatitis [21,22]. Thus, there is a crucial need for novel, preferably non-invasive biomarkers that could be used for the detection of liver diseases such as HCC in order to improve the diagnosis and the management programs for patients at high risk of developing these severe complications associated with HCV infection. miRNAs differential expression and a regulatory role have been observed in liver pathologies, including chronic HCV and HCC [23-29]. Biochemical markers, liver biopsies and imaging techniques, though routinely used, are not very powerful or practical, mainly due to invasiveness or little specificity and sensitivity, especially for HCC.

**Summary of the invention**

Because circulating microRNAs, have been proposed as biomarkers of disease status, the authors investigated whether specific miRNA signatures can be detected in the serum of patients at different stages of chronic HCV disease. Using an effective normalization strategy to reduce variability, the authors assessed the presence of the whole panel of human miRNAs by high-throughput qPCR in individual sera from patients with chronic hepatitis C with or without liver cirrhosis or HCC and the authors compared these miRNA profiles to those obtained from sera of healthy donors or patients with another immune mediated disorder like Cronh's disease.

Twenty one miRNAs were found differentially represented among groups (p<0.05 after multiple testing correction). The levels of miR-122 and miR-885-5p were specifically and consistently increased in the sera of patients with HCV infection compared to healthy controls or patients with Cronh's disease (p<0.001). Moreover, unsupervised and supervised clustering of samples demonstrated that serum miRNAs are good markers of disease progression in HCV-infected patients. The authors further investigated miRNA levels in additional validation groups, revealing that miR-122 and miR-885 showed optimal performance in classifying longitudinal samples before and after the occurrence of HCC. Identification of miR-885-5p putative targets revealed a potential function of this microRNA as modulator of genes important for DNA damage and cell death. Interestingly, *in vitro* transfection of miR-885-5p in hepatoma cell line resulted in increased apoptosis. Serum levels of miR-122 and miR-885-5p are significantly elevated in patients with chronic HCV infection and can be used as blood biomarkers of liver disease progression, in particular HCV-associated liver disease. The authors found that over-expression of miR-885-5p in liver cells favors cell death, so providing a mechanism for the involvement of these miRNAs in HCV disease progression., together with the well-known effect of miRNA-122 on HCV replication.

It is therefore an object of the invention a method for the diagnosis and/or prognosis of a liver disease in a subject or to identify a subject at risk to develop a liver disease comprising the following steps:

> a) measuring the amount of a miR-122 and a miR-885-5p molecule, an equivalent such as a mimic or an isomiR thereof in a biological sample isolated from the subject, and
> b) comparing the measured amount of step a) with an appropriate control amount of a miR-122 and a miR-885-5p molecule, an equivalent such as a mimic or an isomiR thereof,

wherein if the amount of the miR-122 and the mir-885-5p molecule, an equivalent such as a mimic or an isomiR thereof in the biological sample is altered with respect to the control amount, then the subject is affected by a liver disease or is at risk of developing a liver disease.

Preferably the method further comprising measuring the amount of a miR-483-5p molecule, an equivalent such as a mimic or an isomiR thereof and comparing said measured amount of miR-483-5p molecule, an equivalent such as a mimic or an isomiR thereof to an appropriate control amount of miR-483-5pmolecule, an equivalent such as a mimic or an isomiR thereof.

Preferably the liver disease is an HCV-associated liver disease. Still preferably the liver disease is selected from the group consisting of: chronic hepatitis, liver cirrhosis, hepatocellular carcinoma.

In a preferred embodiment the biological sample is a blood or a serum sample.

It is a further object of the invention a method to early diagnosing of hepatocellular carcinoma comprising:

> a) measuring the amounts of a miR-122 and a miR-885-5p molecule, an equivalent such as a mimic or an isomiR thereof in a biological sample isolated from the subject at different times, and
> b) comparing the measured amounts of step a) wherein if the amount of the miR-122 and the mir-885-5p molecule, an equivalent such as a mimic or an isomiR thereof in the biological sample decrease over time, then the subject has an higher risk of developing hepatocellular carcinoma.

It is a further object of the invention a method to monitoring the progression of hepatocellular carcinoma comprising:

> a) measuring the amounts of a miR-122 and a miR-885-5pmolecule, an equivalent such as a mimic or an isomiR thereof in a biological sample isolated from the subject at different times, and
> b) comparing the measured amounts of step a).

It is a further object of the invention a kit for the diagnosis and/or prognosis of a liver disease or to identify a subject at risk to develop a liver diseaseand/or to predict the risk of developing hepatocellular carcinoma in a subject as described above, comprising:

- means to detect and/or measure the amount of miR-122 and miR-885-5p; and optionally
- control means.

Preferably the kit further comprises means to detect and/or measure the amount of miR-483-5p.

It is a further object of the invention a modulator of miR-885-5p for use as a medicament. Preferably the modulator is a nucleic acid molecule. Still preferably the modulator is for use in the treatment and/or prevention of a liver disease. More preferably the liver disease is an HCV-associated liver disease. Still preferably the HCV-associated liver disease is selected from the group consisting of: chronic hepatitis, liver cirrhosis, hepatocellular carcinoma. It is a further object of the invention a pharmaceutical composition comprising at least one modulator as defined above and excipients and/or adjuvants.

In the present invention the modulator of miR-885-5p may be an equivalent such as a mimic, an isomiR or may be an inhibitor.

A miR-885-5p equivalent such as a mimic, an isomiR is characterized as being an oligonucleotide comprising at least 20 nucleotides of any of the sequences SEQ ID No. 25 or an orthologous or a variant thereof.

Variants may also comprise the above mentioned sequences and a modified oligonucleotide conjugated to one or more moieties which enhance the activity, cellular distribution or cellular uptake of the resulting antisense oligonucleotides. A preferred moiety is a cholesterol moiety or a lipid moiety. Additional moieties for conjugation include carbohydrates, phospholipids, biotin, phenazine, folate, phenanthridine, anthraquinone, acridine, fluoresceins, rhodamines, coumarins, and dyes. In certain variants, a conjugate group is attached directly to a modified oligonucleotide. In certain variants, a conjugate group is attached to a modified oligonucleotide by a linking moiety selected from amino, hydroxyl, carboxylic acid, thiol, unsaturations (e.g., double or triple bonds), 8-amino-3,6-dioxaoctanoic acid (ADO), succinimidyl 4-(N-maleimidomethyl) cyclohexane-1-carboxylate (SMCC), 6-aminohexanoic acid (ALEX or AHA), substituted C1-C10 alkyl, substituted or un-substituted C2-C10 alkenyl, and substituted or un-substituted C2-C10 alkynyl. In certain variants, a substituent group is selected from hydroxyl, amino, alkoxy, carboxy, benzyl, phenyl, nitro, thiol, thioalkoxy, halogen, alkyl, aryl, alkenyl and alkynyl. In certain variants, the compound comprises a modified oligonucleotide having one or more stabilizing groups that are attached to one or both termini of a modified oligonucleotide to enhance properties such as, for example, nuclease stability. Included in stabilizing groups are cap structures. These terminal modifications protect a modified oligonucleotide from exonuclease degradation, and can help in delivery and/or localization within a cell. The cap can be present at the 5'-terminus (5'-cap), or at the 3'-terminus (3'-cap), or can be present on both termini. Cap structures include, for example, inverted deoxy abasic caps.

Suitable cap structures include a 4',5'-methylene nucleotide, a 1-(beta-D-erythrofuranosyl) nucleotide, a 4'-thio nucleotide, a carbocyclic nucleotide, a 1,5-anhydrohexitol nucleotide, an L-nucleotide, an alpha-nucleotide, a modified base nucleotide, a phosphorodithioate linkage, a threo-pentofuranosyl nucleotide, an acyclic 3',4'-seco nucleotide, an acyclic 3,4-dihydroxybutyl nucleotide, an acyclic 3,5-dihydroxypentyl nucleotide, a 3'-3'-inverted nucleotide moiety, a 3'-3'-inverted abasic moiety, a 3'-2'-inverted nucleotide moiety, a 3'-2'-inverted abasic moiety, a 1,4-butanediol phosphate, a 3'-phosphoramidate, a hexylphosphate, an aminohexyl phosphate, a 3'-phosphate, a 3'-phosphorothioate, a phosphorodithioate, a bridging methylphosphonate moiety, and a non-bridging methylphosphonate moiety 5'-amino-alkyl phosphate, a 1,3-diamino-2-propyl phosphate, 3-aminopropyl phosphate, a 6-aminohexyl phosphate, a 1,2-aminododecyl phosphate, a hydroxypropyl phosphate, a 5'-5'-inverted nucleotide moiety, a 5'-5'-inverted abasic moiety, a 5'-phosphoramidate, a 5'-phosphorothioate, a 5'-amino, a bridging and/or non-bridging 5'-phosphoramidate, a phosphorothioate, and a 5'-mercapto moiety.

In the context of the invention, a miRNA molecule or an equivalent or a mimic or an isomiR thereof may be a synthetic or natural or recombinant or mature or part of a mature miRNA or a human miRNA or derived from a human miRNA as further defined in the part dedicated to the general definitions. A human miRNA molecule is a miRNA molecule which is found in a human cell, tissue, organ or body fluids (i.e. endogenous human miRNA molecule). A human miRNA molecule may also be a human miRNA molecule derived from an endogenous human miRNA molecule by substitution, deletion and/or addition of a nucleotide. A miRNA molecule or an equivalent or a mimic thereof may be a single stranded or double stranded RNA molecule. Preferably a miRNA molecule or an equivalent, or a mimic thereof is from 6 to 30 nucleotides in length, preferably 12 to 30 nucleotides in length, preferably 15 to 28 nucleotides in length, more preferably said molecule has a length of at least 6, 7, 8, 9, 10, 11, 12, 13, 14, 15, 16, 17, 18, 19, 20, 21, 22, 23, 24, 25, 26, 27, 28, 29, 30 nucleotides or more.

A miRNA molecule or an equivalent or a mimic or an isomiR thereof may have 70 % identity over the whole mature sequence of the miR 885-5p (SEQ ID No. 25 or orthologs thereof), preferably identity is at least 75 %, 80 %, 85 %, 90 %, 95 %, 97%, 98 %, 99% or 100%.

An equivalent of a miRNA molecule may be an isomiR or a mimic. A precursor sequence may result in more than one isomiR sequences depending on the maturation process. A mimic is a molecule which has a similar or identical activity with a miRNA molecule. In this context a similar activity is given the same meaning as an acceptable level of an activity.

Each of the miRNA molecules or equivalents or mimics or isomiRs thereof as identified herein has an acceptable level of an activity of a given miRNA they derive from. An acceptable level of an activity is preferably that said miRNA or equivalent or mimics or isomiRs thereof is still able to exhibit an acceptable level of said activity of said miRNA. An acceptable level of an activity is preferably at least 30%, 40%, 50%, 60%, 70%, 80%, 90%, 95% or 100%, or more than 100%, such as 200% or 300% or more of the activity of the miRNA they derive from.

A source of a miRNA molecule or a source of an equivalent of a miRNA molecule, mimic, isomiR may be any molecule which is able to induce the production of a miRNA molecule or of an equivalent thereof such as a mimic or isomiR as identified herein and which comprises a hairpin-like structure and/or a double stranded nucleic acid molecule. The presence of a hairpin-like structure, may be assessed using the RNA shapes program (Steffen P. et al 2006) using sliding windows of 80, 100 and 120 nt or more. The hairpin-like structure is usually present in a natural or endogenous source of a miRNA molecule whereas a double-stranded nucleic acid molecule is usually present in a recombinant or synthetic source of a miRNA molecule or of an equivalent thereof.

A source of a miRNA molecule or of an equivalent or a mimic or an isomiR thereof may be a single stranded optionally within a hairpin like structure, a double stranded RNA or a partially double stranded RNA or may comprise three strands, an example of which is described in WO2008/10558. As used herein partially double stranded refers to double stranded structures that also comprise single stranded structures at the 5' and/or at the 3' end. It may occur when each strand of a miRNA molecule does not have the same length. In general, such partial double stranded miRNA molecule may have less than 75% double stranded structure and more than 25% single stranded structure, or less than 50% double stranded structure and more than 50% single stranded structure, or more preferably less than 25%, 20 % or 15% double stranded structure and more than 75%, 80%, 85% single stranded structure. Alternatively, a source of a miRNA molecule or of an equivalent or a mimic or an isomiR thereof is a DNA molecule encoding a precursor of a miRNA molecule or of an equivalent or a mimic or an isomiR thereof. The invention encompasses the use of a DNA molecule encoding a precursor of a miRNA molecule that has at least 70% identity with said sequence SEQ ID No. xx. Preferably, the identity is at least 75%, 80%, 85%, 90%, 95%, 97%, 98%, 99% or 100%. Preferably in this embodiment, a DNA molecule has a length of at least 50, 55, 60, 70, 75, 80, 85, 90, 95, 100, 130, 150, 200, 250, 300, 350, 400 nucleotides or more and has at least 70% identity with a DNA sequence as as SEQ ID NO: 6 or 7, variants or orthologs thereof.

The induction of the production of a given miRNA molecule or of an equivalent thereof or of a mimic or an isomiR thereof is preferably obtained when said source is introduced into a cell using one assay as defined below. Cells encompassed by the present invention are later on defined. A preferred source of a miRNA molecule or of an equivalent thereof or of a mimic or an isomiR thereof is a precursor thereof, more preferably a nucleic acid encoding said miRNA molecule or an equivalent thereof or of a mimic or an isomiR thereof. A preferred precursor is a naturally-occurring precursor. A precursor may be a synthetic or recombinant precursor.

A preferred source includes or comprises an expression construct comprising a nucleic acid, i.e. DNA encoding said precursor of said miRNA, more preferably said expression construct is a viral gene therapy vector selected from gene therapy vectors based on an adenovirus, an adeno-associated virus (AAV), a herpes virus, a pox virus and a retrovirus. A preferred viral gene therapy vector is an AAV or lentiviral vector. Other preferred vectors are oncolytic viral vectors. Such vectors are further described herein below. Alternatively, a source may be a synthetic miRNA molecule or a chemical mimic as further defined in the part dedicated to general definitions.

The detection of the presence or of the amount of a miRNA molecule or of an equivalent thereof such as a mimic or an isomiR of a miRNA molecule or equivalent thereof may be carried out using any technique known to the skilled person. The assessment of the expression level or of the presence of such molecule is preferably performed using classical molecular biology techniques such as (real time Polymerase Chain Reaction) qPCR, microarrays, bead arrays, RNAse protection analysis or Northern blot analysis or cloning and sequencing. The skilled person will understand that alternatively or in combination with the quantification of a miRNA molecule or of an equivalent thereof, the quantification of a substrate of a corresponding miRNA molecule or of an equivalent thereof of any compound known to be associated with a function of said miRNA molecule or of said equivalent thereof or the quantification of a function or activity of said miRNA molecule or of said equivalent thereof using a specific assay is encompassed within the scope of the invention.

A miRNA molecule or an equivalent thereof or a mimic or an isomiR thereof may be used as such as a naked molecule, with or without chemical modifications, or encapsulated into a particle or conjugated to a moiety. A preferred composition comprises a miRNA molecule or an equivalent thereof or a mimic or an isomiR thereof encapsulated into a nanoparticle or a liposomal structure. A miRNA molecule or equivalent thereof or a mimic or an isomiR thereof may be an aptamer-miRNA hybrid. An aptamer-miRNA is defined as a miRNA linked to an RNA (or DNA) oligonucleotide, the latter adopting a conformation that targets the aptamer-miRNA hybrid molecule to a cell-surface protein (e.g. cyclic RGD peptide (cyclic arginine(R)-glycine(G)-aspartic acid(D) peptide). The aptamer-tagged miRNA can be linked to e.g. polyethylene glycol, which increases the chimera's circulating half-life (Dassie, J.P., et al. 2009).

In the present invention, miR inhibitors are single-stranded, chemically modified nucleic acid molecules that regulate gene expression by binding to and inhibiting a specific mature miRNA. Preferably they are small nucleic acid molecules. In the present invention miR inhibitors and/or antagomirs are chemically engineered oligonucleotides which are used to silence endogenous microRNAs. An antagomir is a small synthetic RNA that is perfectly complementary to the specific miRNA target with either mispairing at the cleavage site of Ago2 or some sort of base modification to inhibit Ago2 cleavage. Usually, antagomirs have some sort of modification to make it more resistant to degradation. It is unclear how antagomirization (the process by which an antagomir inhibits miRNA activity) operates, but it is believed to inhibit by irreversibly binding the miRNA. Antagomirs are used to constitutively inhibit the activity of specific miRNAs.

The preferred variants of the antagomir sequence of SEQ ID NO:25 are at least 85%, preferred 90% and more preferred 95% homologue to the SEQ ID NO: 25. This means that in the variants up to three nucleotides can be replaced by other nucleotides, preferably only two and more preferred only one nucleotide is replaced. The term homology is understood as identity. This means that e.g. at least 85% of the nucleotides are identical whereas the remainder of the nucleotides may be changed.

The biologically active structures as described herein can be introduced into the body of the person to be treated as a nucleic acid within a vector which replicates into the host cells and produces the oligonucleotides.

Variants may also comprise the above mentioned sequences and a modified oligonucleotide conjugated to one or more moieties which enhance the activity, cellular distribution or cellular uptake of the resulting antisense oligonucleotides. A preferred moiety is a cholesterol moiety or a lipid moiety. Additional moieties for conjugation include carbohydrates, phospholipids, biotin, phenazine, folate, phenanthridine, anthraquinone, acridine, fluoresceins, rhodamines, coumarins, and dyes. In certain variants, a conjugate group is attached directly to a modified oligonucleotide. In certain variants, a conjugate group is attached to a modified oligonucleotide by a linking moiety selected from amino, hydroxyl, carboxylic acid, thiol, unsaturations (e.g., double or triple bonds), 8-amino-3,6-dioxaoctanoic acid (ADO), succinimidyl 4-(N-maleimidomethyl) cyclohexane-1-carboxylate (SMCC), 6-aminohexanoic acid (ALEX or AHA), substituted C1-C10 alkyl, substituted or un-substituted C2-C10 alkenyl, and substituted or un-substituted C2-C10 alkynyl. In certain variants, a substituent group is selected from hydroxyl, amino, alkoxy, carboxy, benzyl, phenyl, nitro, thiol, thioalkoxy, halogen, alkyl, aryl, alkenyl and alkynyl. In certain variants, the compound comprises a modified oligonucleotide having one or more stabilizing groups that are attached to one or both termini of a modified oligonucleotide to enhance properties such as, for example, nuclease stability. Included in stabilizing groups are cap structures. These terminal modifications protect a modified oligonucleotide from exonuclease degradation, and can help in delivery and/or localization within a cell. The cap can be present at the 5'-terminus (5'-cap), or at the 3'-terminus (3'-cap), or can be present on both termini. Cap structures include, for example, inverted deoxy abasic caps. Suitable caps are indicated above.

Since miRNAs target their mRNA by Watson-Crick base-pairing it is preferred that the inhibitor of miR 885-5p is an antisense oligonucleotide, which is complementary to the miRNA and base pairs with the miRNA in competition with the endogenous mRNA target. For the purpose of the invention, the sequence of the antisense oligonucleotide is 50% identical to the complement of miR 885-5p or its seed sequence, preferably 60%, 70%, 80%, 90%, or 95% and most preferably 100% identical to the complement of the miR 885-5p and/or its seed sequence. Moreover, particularly preferred are antisense oligonucleotides which are chemically modified to improve the thermal stability of the duplex between the antisense oligonucleotide and the miRNA. Preferred chemical modifications comprise, for example, bicyclic high-affinity RNA analogues in which the furanose ring in the sugar-phosphate backbone is chemically locked in an RNA mimicking N-type conformation by the introduction of 2'-O,4'-C-methylene bridge (LNA(R)-antimiRs). Other preferred chemical modified oligonucleotides include morpholinos, 2'-O-methyl, 2'-O-methoxyethyl oligonucleotides and cholesterol-conjugated 2'-O-methyl modified oligonucleotides (antagomirs).

Inhibitors in context of the invention also comprise any substance that is able to inhibit miR 885-5p either by inhibiting the expression or by inhibiting the silencing function of the microRNA. Thus, any compound interfering with the microRNA pathway, for example by inhibiting the function of the proteins Pasha, Drosha, Dicer or Argonaut family proteins can be an antagonist according to the invention. Furthermore any compound inhibiting the expression of the precursor microRNA of miR 885-5p, such as, for example inhibitors of polymerase II or III are candidate antagonists of miRNA expression. The mature miRNA also serves as a target for the design of inhibitors of miR 885-5p function. Nucleic acids having perfect or mismatched complementarity to the microRNA may be used to inhibit, or to compete with the binding of the endogenous miR 885-5p with its target mRNA. How to design such miRNA inhibitors is well known in the art.

Inhibitors of miR 885-5p, modified oligonucleotide complementary to a miR 885-5p, or precursor thereof, described herein may be prepared as a pharmaceutical composition. In particular for the treatment of liver diseases. Suitable administration routes include, but are not limited to, oral, rectal, transmucosal, intestinal, enteral, topical, suppository, through inhalation, intrathecal, intraventricular, intraperitoneal, intranasal, intraocular and parenteral (e.g., intravenous, intramuscular, intramedullary, and subcutaneous). An additional suitable administration route includes chemoembolization. In certain embodiments, pharmaceutical intrathecals are administered to achieve local rather than systemic exposures. For example, pharmaceutical compositions may be injected directly in the area of desired effect.

In certain embodiments, a pharmaceutical composition of the present invention is administered in the form of a dosage unit (e.g., tablet, capsule, bolus, etc.).

The inhibitor or the miRNA 885-5p molecule, an equivalent such as a mimic, an isomiR or a source thereof may be administered as a pharmaceutical composition comprising a pharmacologically acceptable carrier and diluent. Administration may be carried out by known methods, wherein the inhibitor is introduced into the desired target cell in vitro or in vivo. Suitable administration methods include injection, viral transfer, use of liposomes, e.g. cationic liposomes, oral intake and/or dermal application.

For pharmaceutical applications, the composition may be in the form of a solution, e.g. an injectable solution, emulsion, suspension or the like. The composition may be administered in any suitable way, e.g. by injection, infusion, oral intake and/or by dermal application. The carrier may be any suitable pharmaceutical carrier. Preferably, a carrier is used which is capable of increasing the efficacy of the RNA molecules to enter the target cells. Suitable examples of such carriers are liposomes.

The inhibitor or the miR-885-5p molecule, an equivalent such as a mimic, an isomiR or a source thereof is administered in a pharmaceutically effective dosage, which may be in the range of 0.001 pg/kg body weight to 1 mg/kg body weight depending on the route of administration and the type or severity of the disease.

The inhibitor of the present invention may comprise a single type of inhibitor molecule or a plurality of different inhibitor molecules, e.g. a plurality of different siRNA molecules and/or antagomirs. For example, an inhibitor of miR-885-5p, e.g. an antagomir, may be combined with an inhibitor of another miR.

The inhibitor or the miR-885-5p molecule, an equivalent such as a mimic, an isomiR or a source thereof may be administered as a monotherapy or in combination with a further different medicament, particularly a medicament suitable for the prevention and/or treatment of liver diseases as described above.

The present invention will be illustrated by means of non-limiting examples in reference to the following figures.

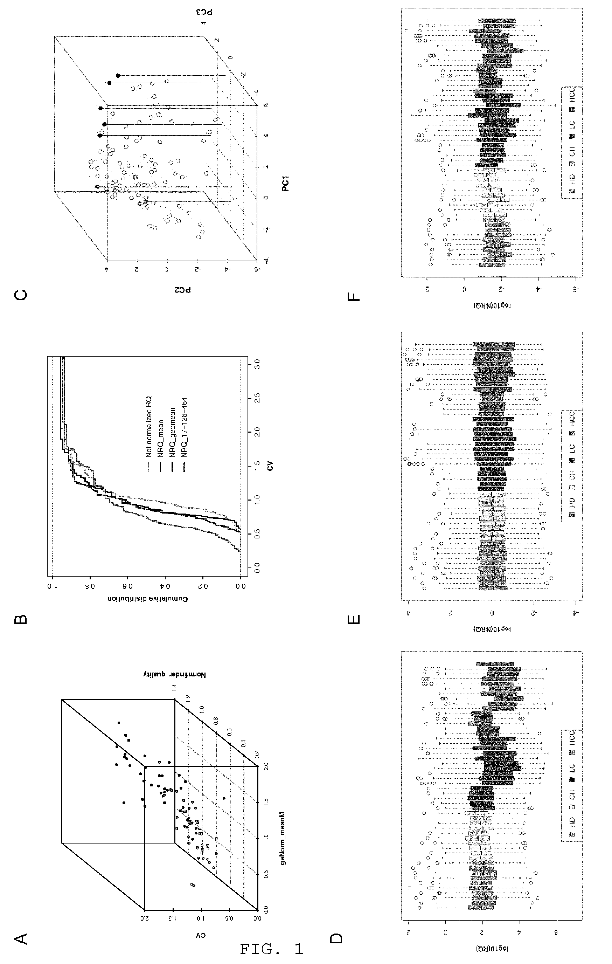

**Figure 1. Selected circulating miRNAs effectively reduce data variability and can be used as endogenous controls. (A)** A three-dimensional scatter plot shows the scores resulting from the three algorithms used for the selection of reference miRNAs. miRNAs with the lowest scores are the more stable. Normalized Relative Quantities (NRQs) were obtained using the global geometric or arithmetic mean of all expressed miRNAs per sample or alternatively using the geometric mean of the three top reference miRNAs (miR-17, miR-484, miR-126). **(B)** The effective reduction in data variability was evaluated looking at the cumulative distribution of the coefficient of variation (CV) when data were not normalized (grey line) or normalized (yellow, blue and red lines). (C) The first three principal components of the autoscaled RQs are shown in a 3D scatterplot. miR-17, miR-126 and miR-484 are shown as red dots, while mir-21, miR-122, miR-483-5p, miR-610 ad miR-885-5p are shown as black dots. **(D, E and F)** The distributions of miRNA levels in samples is shown as a boxplot, in RQ or NRQ units. Data were normalized with the global geometric mean **(E)** and with the reference miRNAs **(F),** respectively. Samples are coded as follows: green for healthy Donors (HD), yellow for chronic hepatitis (CH), blue for liver cirrhosis (LC) and red for hepatocellular carcinoma (HCC).

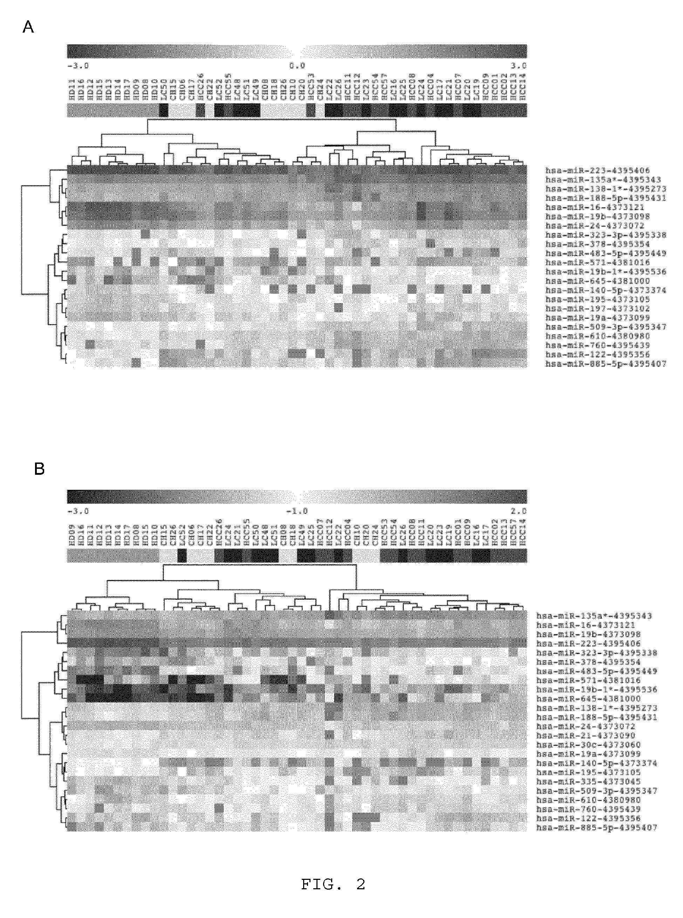

**Figure 2. Circulating miRNAs are differentially represented in the serum of healthy donors and patients with chronic HCV infection.** Differentially represented miRNAs after ANOVA on miRNAs detectable in at least two thirds of samples per group (p<0.05 after Bonferroni correction). Blue denotes low expression, white intermediate expression and red high expression, in logarithmic scale. All values are relative quantities normalized with **(A)** the global sample geometric mean or **(B)** the geometric mean of mir-17, miR-126 and miR-484. Samples are coded as follows: green for healthy Donors (HD), yellow for chronic hepatitis (CH), blue for liver cirrhosis (LC) and red for hepatocellular carcinoma (HCC). Hierarchical clustering with Euclidean distance and complete linkage was used.

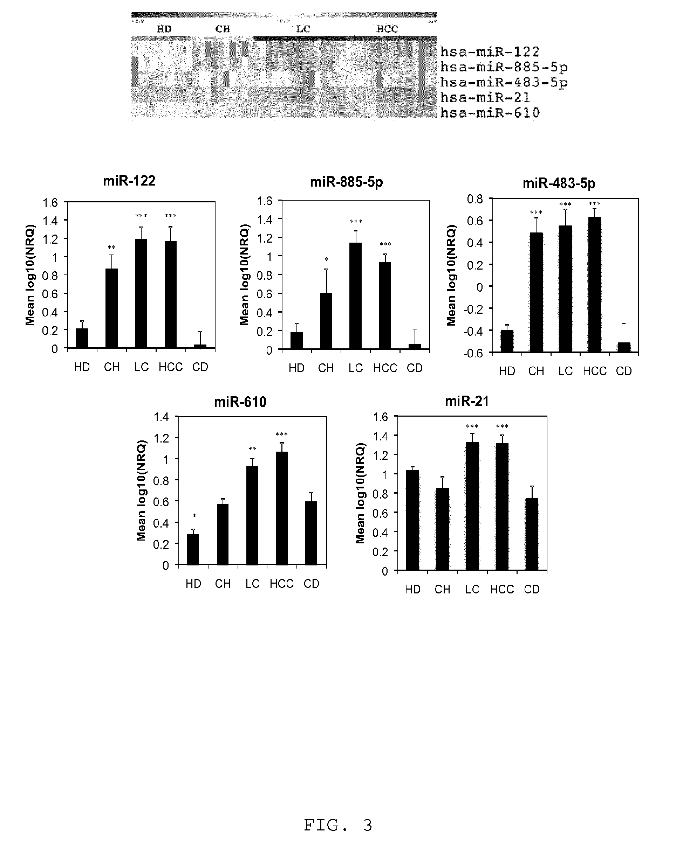

**Figure 3. miR-122, miR-483-5p and miR-885-5p are specifically and consistently increased in the serum of patients with chronic HCV infection, correlating to disease. (A)** A heat map of miRNAs correlated to miR-122 (R>0.6) is shown. Color codes are the same as in figure 2. **(B)** Bar plots of miR-122, miR-483-5p, miR-885-5p, miR-21 and miR-610. Black bars show the mean log10(NRQ) plus the SEM. Only miR-122, miR-483-5p and miR-885-5p are specific hallmark of chronic liver inflammation, compared to the control groups of CD patients. Abbreviations: HD, Healthy donors; CH, chronic hepatitis; LC, liver cirrhosis; HCC, hepatocellular carcinoma; CD, Crohn's disease. Significant results are compared to CD and are indicated by: *, p<0.05; **, p<0.01, ***, p<0.001.

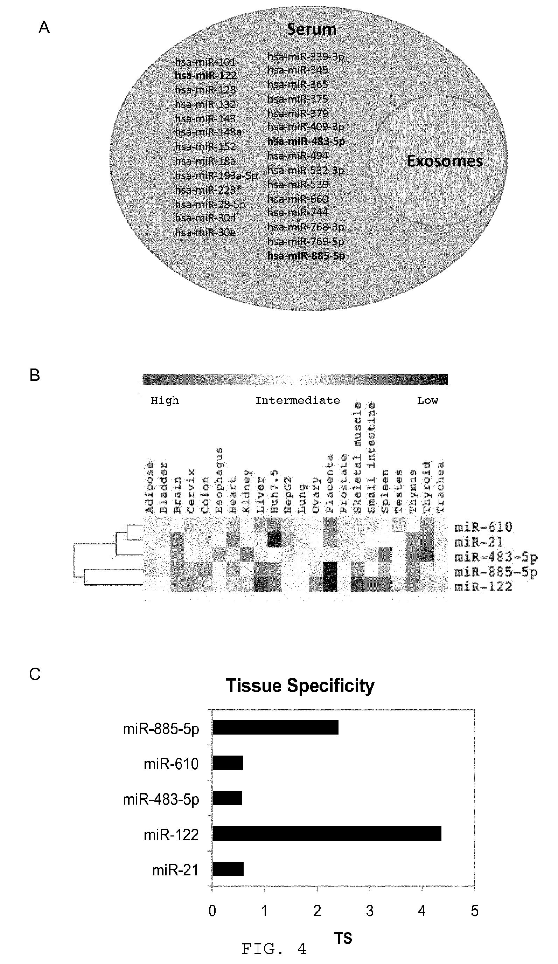

**Figure 4. miR-122 and miR-885-5p are enriched in the liver and are non-exosome associated in the blood. (A)** The whole miRNome was profiled by TaqMan Arrays, from four sera and from four corresponding exosome preparation, obtained from four healthy donors. The analysis shows that miR-122, miR-483-5p and miR-885-5p are among the miRNAs that are detectable only in serum and not in the exosome preparation. These miRNAs were defined as those having a Ct ≤ 30 in at least 3 out of 4 samples in serum specimens and a Ct ≥ 35 in in at least 3 out of 4 samples in exosome specimens. **(B)** The expression of the indicated miRNAs in different tissues is shown after detection with single TaqMan assays and normalization with snRNA U6. Each miRNA was further normalized to its median ΔCt values across samples, hence the data reflect the relative expression among tissues. (C) The tissue specificity scores (TS) reveals that onlty miR-122 and miR-885-5p may be actually considered as tissue-restricted. TS were calculated as the relative entropy for each miRNA.

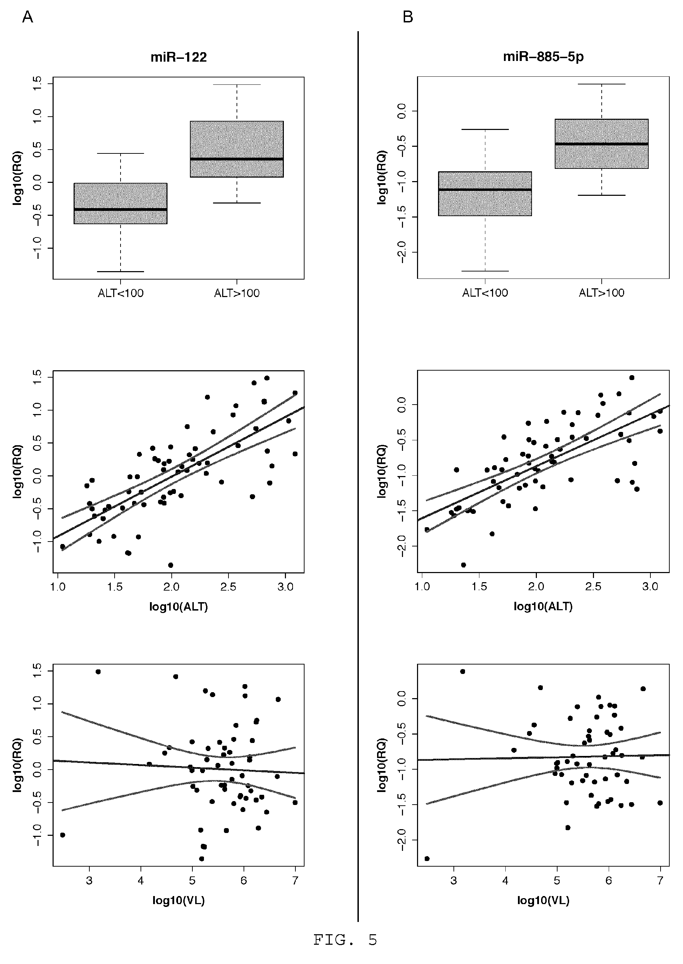

**Figure 5. miR-122 and miR-885-5p levels correlated with alanine aminotransferase (ALT) activity. (A)** miR-122 and **(B)** miR-885-5p Relative Quantities (RQs) were calculated by single TaqMan qPCR assays. Note that the scale on the y-axis is different from those in figures 1-3, as a result of different platform and different normalization. The concentrations changed significantly among groups with different ALT activity both for miR-122 (p=7.5E-09) and miR-885-5p (p=1.13E-07). miRNA levels positively correlated with ALT activity (miR-122 p=8.65E-12 and miR-885-5p p=2.42E-10) but not with viral load (VL, p>0.05). The best fit line (in blue) plus the 95% confidence interval (in red) are shown.

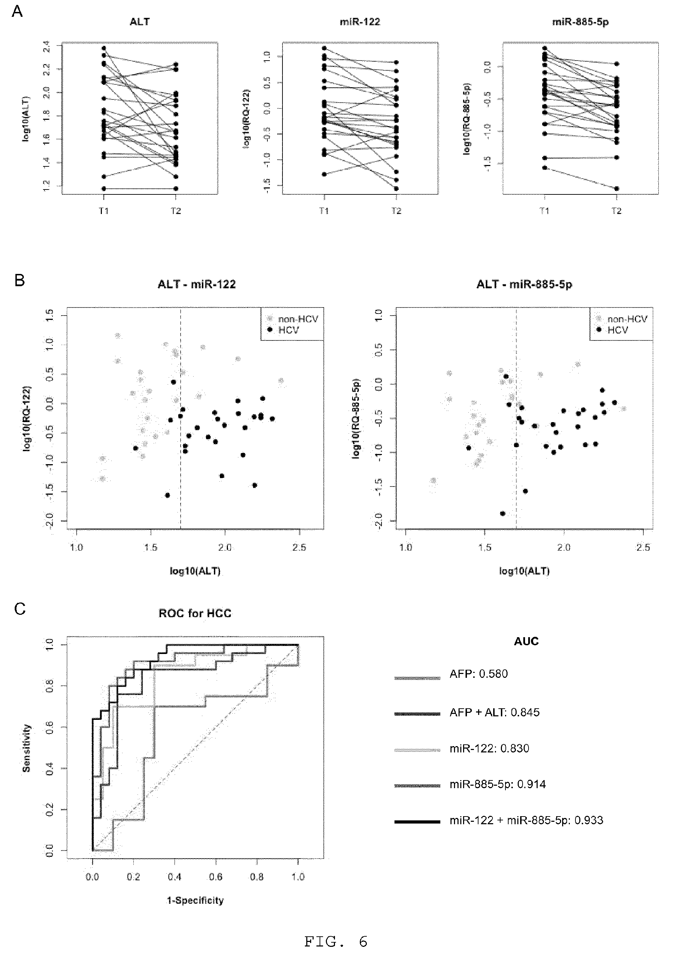

**Figure 6. Circulating miR-122 and miR-885-5p are decreased after HCC occurrence and are best disease predictors.** miRNA Relative Quantities (RQs) were calculated by single TaqMan qPCR assays on paired samples before (T1) and after (T2) the occurrence of HCC. Note that the scale on the y-axis is different from those in figures 1-3, as a result of different platform and different normalization. **(A)** miR-122 (p=7.66E-03) and miR-885-5p (p=1.22E-04) and ALT activity (p=0.0149) decreased significantly. **(B)** A paired linear model analysis revealed that there is no significant correlation between ALT and miRNA levels, but two different clusters of samples are highlighted, discriminating patient with HCV etiology vs. non-HCV etiology. The dashed vertical line indicates the normal limit for ALT (50 IU/l) **(C)** A paired logistic regression model was used to predict the HCC class and the performance of different models was evaluated using ROC curve analysis. The combination of miR-122 and miR-885-5p showed the best performance, as indicated by the AUC (area under the curve).

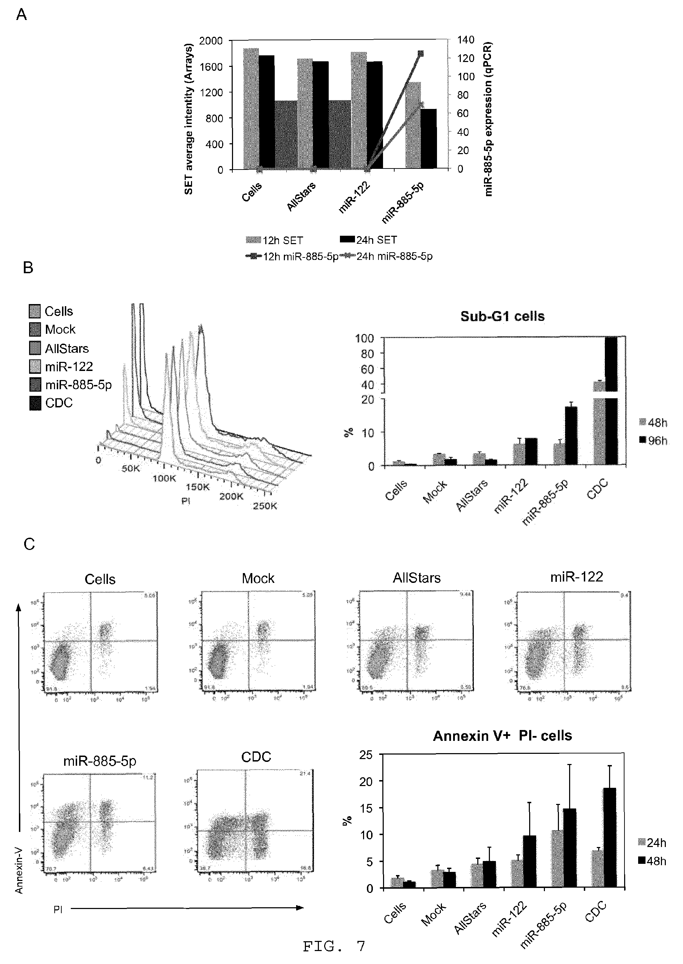

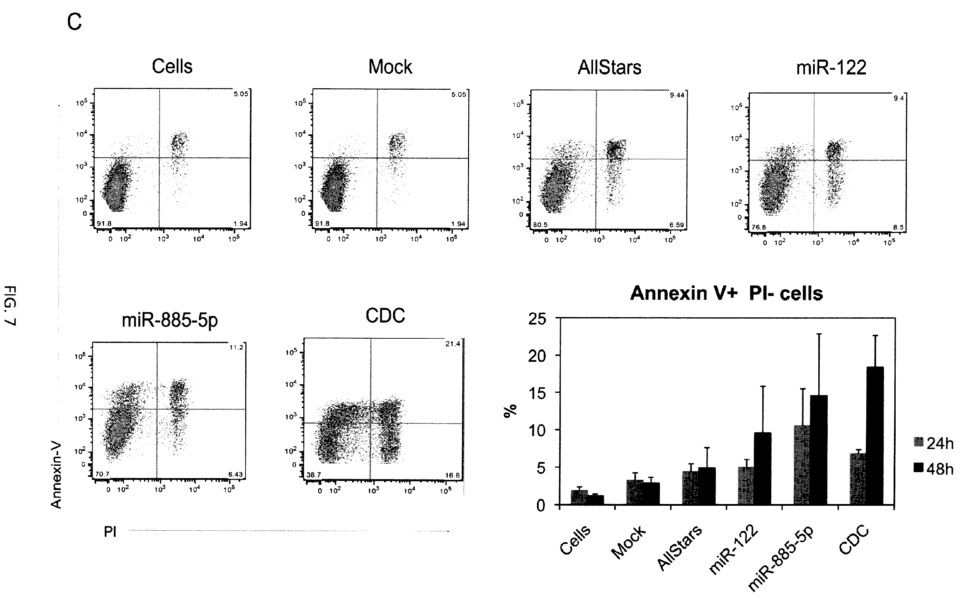

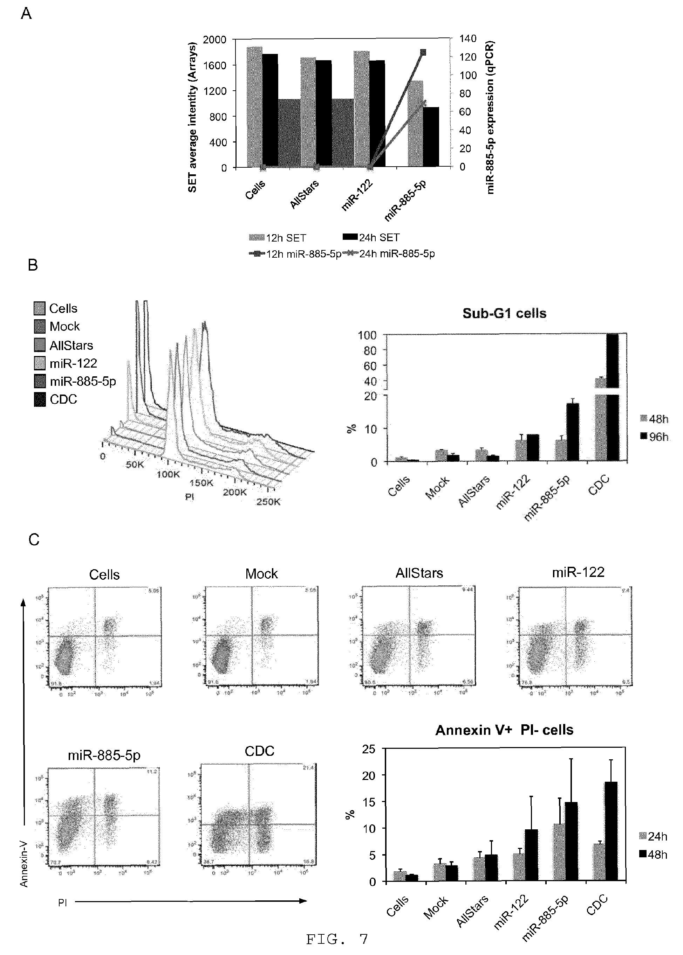

**Figure 7. miR-885-5p as modulator of cell death in hepatoma cell line. (A)** After miR-885-5p transfection, gene expression analysis revealed that the SET transcript was negatively modulated and reduced to 78% and 55%, twelve and twenty-four hours post-transfection respectively, compared to AllStars negative control. **(B)** PI staining and FACS analysis of DNA content 96 hrs post transfection revealed the presence of hapodyploid DNA content (sub-G1 peaks). The histogram shows the mean value + SEM of three experiments. **(C)** Phosphatidylserine externalization 48 hrs was assessed by AnnevinV/PI staining. The histogram shows the mean value + SEM of three experiments. Abbreviation: Cells, untransfected cells; Mock, cells with transfection reagent only; AllStars, cells transfected with negative control; miR-122, cells transfected with miR-122; miR-885-5p, cells transfected with miR-885-5p; CDC, cell death positive control siRNA.

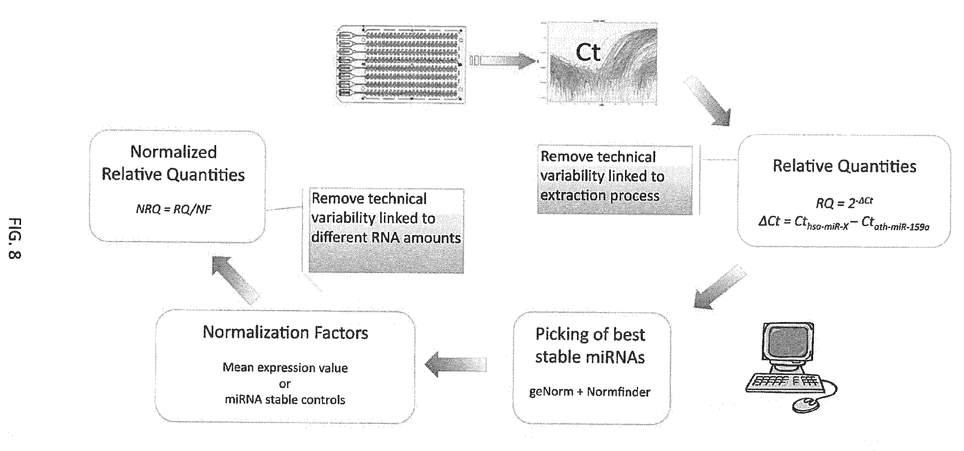

**Figure 8.** The workflow of the normalization process. Definition are given within the text.

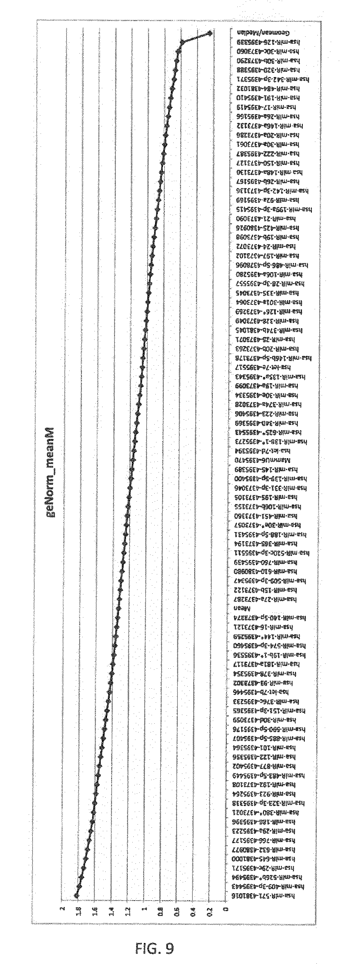

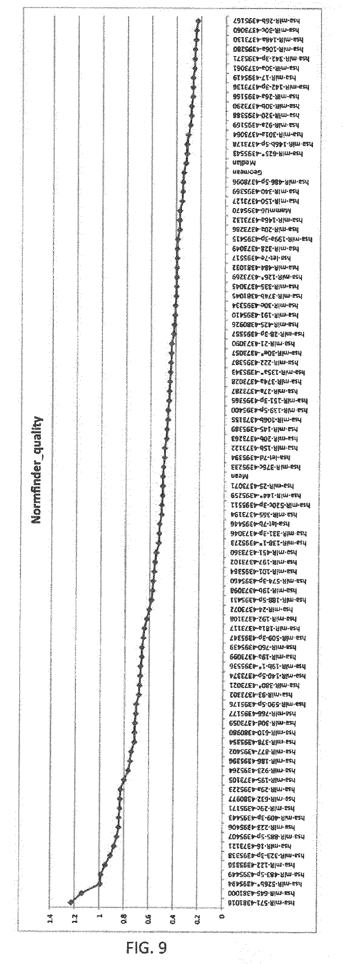

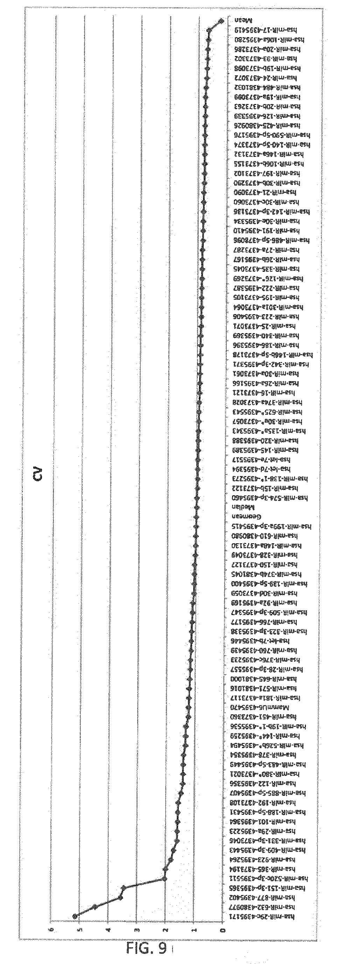

**Figure 9. The** scores associated with the tested miRNAs using three algorithms to detect stable reference miRNAs. A lower score correspond to better stability.

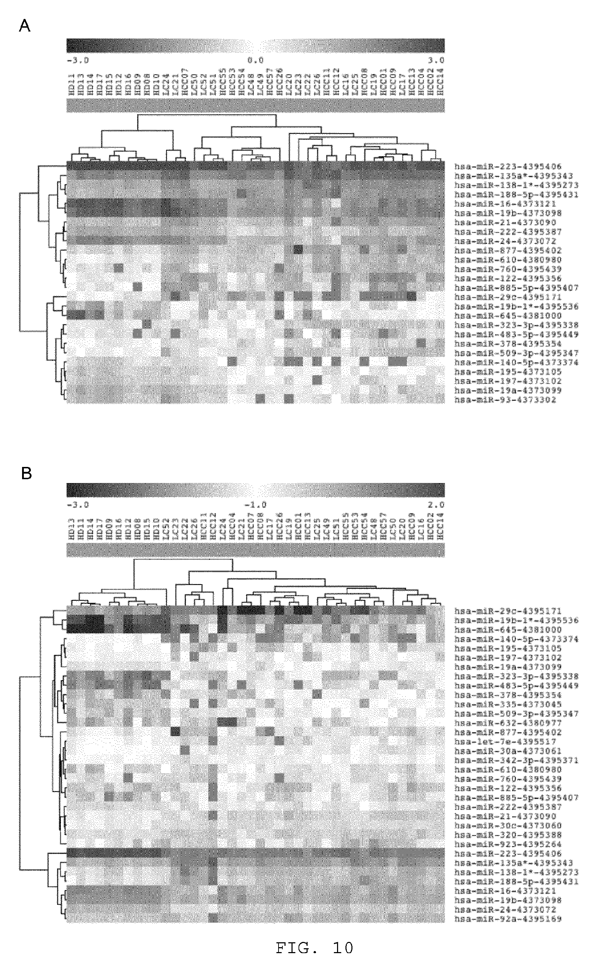

**Figure 10.** Differentially represented miRNAs (p<0.05, t-test with Bonferroni correction) between Healthy Donors (HD) hepatocellular carcinoma (HCC) samples. Hierarchical clustering with Euclidean distance and complete linkage was used.

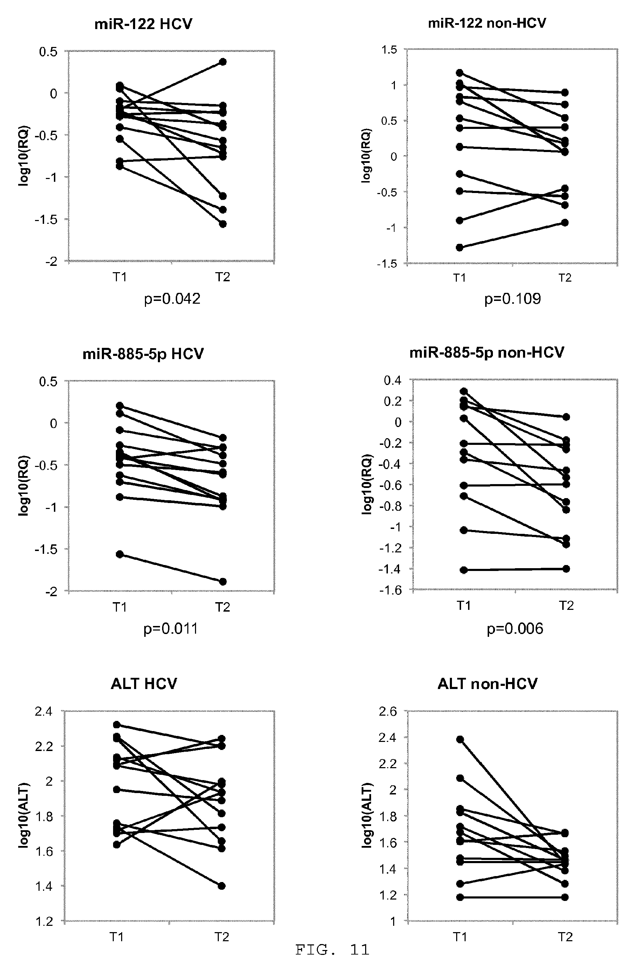

**Figure 11**. Levels of miR-122, miR-885-5p and ALT before (T1) and after (T2) the occurrence of HCC. Sample are stratified according to the disease etiology (HCV vs non-HCV)

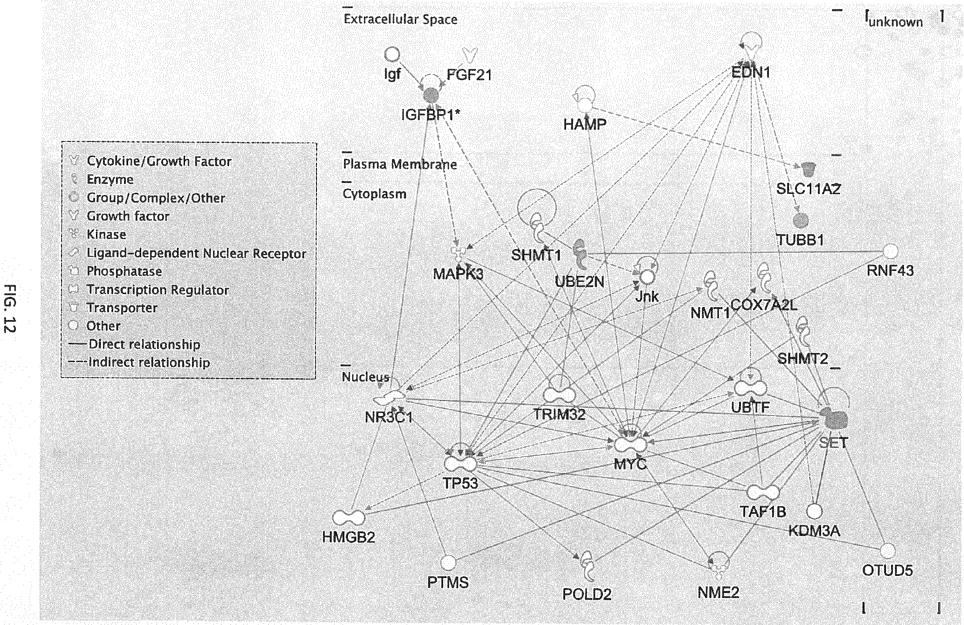

**Figure 12.** Network generated by Ingenuity Pathway analysis using the miR-885-5p target inversely correlated to miRNA expression (see main text). Direct down-regulated target are shown in green.

## Detailed description of the invention

### Methods

### Patients characteristics

Frozen human sera from were obtained from U.O. Epatologia, Azienda Ospedaliera Universitaria Pisana (Pisa). A Discovery Group consisted in 40 patients with chronic HCV infection and included: 10 patients with chronic hepatitis but without liver cirrhosis or HCC (hereafter CH), 15 patients with liver cirrhosis (hereafter LC) and 15 patients with liver cirrhosis and HCC (hereafter HCC). The authors included also Validation Group I and II in the analysis. Details about the clinical characteristics are given in Tables 1 - 3.

**Table1.** Clinical characteristics of Discovery Group

| | Healthy Donors (HD) n=10 | Chronic Hepatitis (CH) n=10 | Liver Cirrhosis (LC) =15 | Hepatocellular Carcinoma (HCC) n=15 |
|---|---|---|---|---|
| **Sex - No. (%)** | | | | |
| Male | 9 (90%) | 5 (50%) | 10 (67%) | 9 (60%) |
| Female | 1 (10%) | 5 (50%) | 5 (33%) | 6 (40%) |
| **Age - years** | | | | |
| Mean (SD) | 42.2 (8.2) | 52 (17) | 60.7 (7.4) | 71.6 (9.8) |
| Median (range) | 37.5 (33 - 58) | 48 (25 - 78) | 63 (47 - 70) | 77 (62 - 87) |
| **ALT - No. (%)** | | | | |
| High, > 50 IU/l | - | 8 (80%) | 11 (73%) | 13 (87%) |
| Normal, < 50 IU/l | 10 (100%) | 2 (20%) | 4 (27%) | 2 (13%) |
| **Tumor diameter - No. (%)** | | | | |
| ≤ 3 cm | - | - | - | 10 (66%) |
| > 3 cm | - | - | - | 5 (33%) |
| **Multinodularity - No. (%)** | | | | |
| No | - | - | - | 11 (73%) |
| Yes | - | - | - | 4 (27%) |
| **HCV genotype - No. (%)** | | | | |
| 1 | - | 4 (40%) | 9 (60%) | 10 (66%) |
| 2 | - | 5 (50%) | 2 (13%) | - |

(continued)

|  | *Healthy Donors (HD) n=10* | *Chronic Hepatitis (CH) n=10* | *Liver Cirrhosis (LC) =15* | *Hepatocellular Carcinoma (HCC) n=15* |
|---|---|---|---|---|
| 3 | - | 1 (10%) | 2 (13%) | - |
| Unknown | - | - | 2 (13%) | 5 (33%) |
| **HBV exposure - No. (%)** |  |  |  |  |
| Yes | - | 1 (10%) | 6 (40%) | 6 (40%) |
| No | 10 (100%) | 6 (60%) | 7 (47%) | 6 (40%) |
| Unknown | - | 3 (30%) | 2 (13%) | 3 (20%) |

**Table 2.** Clinical characteristics of Validation Group I

|  | *Acute Hepatitis n=20* | *Chronic Hepatitis n=27* | *Liver Cirrhosis n=10* | *Hepatocellular Carcinoma n=10* |
|---|---|---|---|---|
| **Age - years** Median (range) | 57 (37 - 69) | 46 (25 -78) | 61 (43 - 68) | 69 (47 - 87) |
| **ALT - IU/l** Median (range) | 346 (11-1213) | 98 (18 -760) | 54.5 (20 - 232) | 82.5 (26 - 174) |
| **Viral load -** Median (range) | 5.3E05 (3.0E02 - 1.3E06) | 4.2E05 (2.9E04 - 9.8E06) | 6.2E05 (1.5E04 - 2.2E06) | 4.1E05 (4.7E04 - 1.3E06) |
| **HCV genotype - No. (%)** |  |  |  |  |
| 1 | 10 | 11 | 4 | 6 |
| 2 | 6 | 14 | 3 | - |
| 3 | 2 | 2 | 3 | - |
| Unknown | 2 | - | - | 4 |
| **Tumor diameter - No. (%)** |  |  |  |  |
| ≤ 3 cm | - | - | - | 6 (60%) |
| > 3 cm | - | - | - | 4 (40%) |

**Table 3.** Clinical characteristics of Validation Group II

|  | *HCV etiology (n=26)* |  | *non-HCV etiology (n=24)* |  |
|---|---|---|---|---|
|  | *T1 (n=13)* | *T2 (n=13)* | *T1 (n=12)* | *T2 (n=12)* |
| **Age - years** Median (range) | 61 (35 - 73) | 65 (37 - 81) | 60 (50 - 77) | 65 (55 - 81) |
| **ALT - IU/l** Median (range) | 105.5 (43 - 208) | 81 (25 - 174) | 44 (15 - 239) | 28.5 (15 - 47) |
| **AFP-ng/ml** Median (range) | 12.4 (5.1 - 238.5) | 10.95 (4.49 - 551) | 10.3 (1.6 - 11.6) | 4.9 (1.21 - 519) |

(continued)

| | HCV etiology (n=26) | | non-HCV etiology (n=24) | |
|---|---|---|---|---|
| | T1 (n=13) | T2 (n=13) | T1 (n=12) | T2 (n=12) |
| **Tumor diameter - No. (%)** | | | | |
| ≤ 3 cm | - | 9 (75%) | - | 10 (83%) |
| > 3 cm | - | 5 (25%) | - | 2 (17%) |

Additionally, a group of sera from 10 healthy donors (HD) and 12 patients with Crohn's disease during active phase (CD) were included in the study as controls. These sera were respectively obtained from U.O. Centro Trasfusionale and U.O. Gastroenterologia, Ospedale Maggiore di Milano.

**RNA extraction from sera samples**

Total RNA was extracted from human serum using miRVana miRNA isolation kit (Ambion), as specified in the protocol, with some modifications. Briefly, 70 μl of thawed serum were mixed with 530 μl of lysis solution composed of RNA Lysis Buffer and synthetic ath-miR-159a (final concentration 5 pM). This miRNA was used as process control, for technical normalization. To reduce sample-to-sample variation in quantification of ath-miR-159a, a mix of RNA Lysis Buffer and ath-miR-159a was prepared at the moment and then mixed to the sera being processed. RNA was extracted with acid phenol-chloroform, then further purified with glass-fiber columns and finally eluted in 50 μl of nuclease-free water. RNA was then stored at -80 °C.

**miRNA profiling**

3 μl of total RNA were processed for Reverse Transcription and Preamplification with Megaplex Primer Pools A v2.1 and B v2.0 (Applied Biosystems), according to manufacturer instruction. TaqMan Low Density Arrays (Applied Biosystems) were run on a 7900HT Fast Real-Time PCR System. A total of 664 human miRNAs, 6 human small RNA and 1 control miRNA from *A. Thaliana* were profiled in parallel. Ct values were extracted using RQ Manager, setting a manual threshold of 0.06.

**Analysis workflow and definitions**

Figure 8 shows the analytical workflow used throughout this study. Quantities are defined in this section. Relative Quantities (RQs) are those resulting after scaling Ct values with the external reference ath-miR-159a and moving to the linear scale:

$$RQ = 2^{-\Delta Ct}$$
$$\Delta Ct = Ct_{miRNA} - Ct_{ath-miR-159a}$$

The Normalization Factor (NF) is calculated as the geometric mean of the selected k normalizers, for each sample *j*. The NF is used to obtain the matrix of Normalized Relative Quantities (NRQ) for each miRNA i and sample *j*, starting from RQ:

$$NRQ_{ij} = \frac{RQ_{ij}}{NF_j}$$

Alternatively, NRQ may be obtained using a NF resulting from the arithmetic or geometric mean of all expressed miRNAs per sample, i.e. the mean obtained omitting detectors whose Ct is undetermined (Ct = 40).

**Selection of reference miRNAs**

Three different algorithms were used to reveal the best internal reference miRNAs, namely geNorm,[30] Normfinder[31] and a scoring on the basis of the Coefficient of Variation (CV score). Outliers resulting from artificial, non-exponential amplifications were identified and removed after visual inspection of the amplification curves. A list containing the miRNAs detected in at least two third of samples per group, plus the arithmetic mean, the geometric mean and the median value of RQs was used as input. Missing values were imputed using the impute library (http://CRAN.R-project.org/package=impute) and geNorm was run with the SLqPCR library (http://www.bioconductor.org/packages/2.8/bioc/html/SLqPCR.html) in R. The CV score algorithm is described as follows: from the matrix of RQ (m detectors, n samples), calculate a new matrix **X** for miRNA i and sample *j* where:

$$X_{ij} = \frac{RQ_{ij}}{\sum\limits_{i=1}^{n} RQ_{ij}}$$

Then, for each miRNA i, calculate the CV of $X_{i*}$. This parameter is useful to evaluate stability across samples. Actually the sum of all RQ for each sample *j* can be considered a surrogate of the total miRNA amount. As a consequence, the lesser the CV of $X_{i*}$, the better the miRNA i may be considered as normalizer. By definition, a normalizer must be correlated to the total miRNA amount.

The three algorithms output a score that represents the stability of the candidate miRNA in the expression dataset, in such a way that a smaller score corresponds to higher expression stability. The top candidate miRNAs were selected among those performing well in all three methods. To summarize the results, the distance from the origin in 3-dimensional space was used as a metric:

$$Distance = \sqrt{\left(Score_{geNorm}\right)^2 + \left(Score_{Normfinder}\right)^2 + \left(Score_{CV}\right)^2}$$

Ranking distances in ascending order, the final miRNA reference list was obtained. Principal Component Analysis (PCA) was exploited as an independent method to look at variation in RQ. The input list of miRNAs was submitted to PCA, after $log_{10}$-transformation and imputation of missing data. Autoscaled RQs for miRNA i and sample *j* are defined as follows:

$$ARQ_{ij} = \frac{\log_{10}(RQ_{ij}) - \overline{\log_{10}(RQ_{i*})}}{st.\,dev(\log_{10}(RQ_{i*}))}$$

where $\log_{10}(RQ_{i*})$ and *st. dev*($\log_{10}(RQ_{i*})$) are the mean and the standard deviation of $\log_{10}$(RQ), respectively. Autoscaled data have mean zero and standard deviation of one.

**Differential expression analysis and classification**

Data were loaded on MeV (v4.5) for testing differential expression and clustering. For two-class data, unpaired T-test was used to assess differential expression. Alternatively, ANOVA was used for multi-class data. Data were considered significant if Bonferroni-corrected p<0.05. Hierarchical clustering (HCL) was performed on samples and significant miRNAs, using Euclidean distance and complete linkage. Predictive Analysis of Microarrays (PAM) and Support Vector Machine (SVM) algorithms were applied in R on scaled data, using the packages pamr (http://CRAN.R-project.org/package=pamr) and e1071 (http://CRAN.R-project.org/package=e1071). respectively.

**Isolation of exosomes**

Exosomes were purified by differential centrifugation as described previously[32]. Briefly, 9 ml of serum were diluted with PBS and centrifuged for 30 min at 2,000 × g, for 45 min at 12,000 × g and for two hours at 110,000 × g. The pellet was resuspended in PBS, filtered through a 0.22 μm filter, centrifuged for 70 min at 110,000 × g and washed once in PBS for 70 min at 110,000 × g. RNA was extracted as specified above and of 3 μl were used for profiling with TaqMan arrays.

**qPCR**

Five $\mu$l of total RNA from serum samples were processed for Reverse Transcription using the TaqMan miRNA Reverse Transcription Kit and miRNA-specific stem-loop primers (Applied Biosystems) according to manufacturer's instructions. For real-time PCR, 1 $\mu$l of RT product were combined with TaqMan Universal PCR Master Mix, No AmpErase and TaqMan miRNA Assay (Applied Biosystem) in 15 $\mu$l of final volume. $\Delta$Ct values were calculated using the mean of miR-17, miR-126 and miR-484 Ct values as reference. To profile the miRNA expression in a panel on human tissues or cultured cells, 10 ng of commercial RNA were processed for RT (FirstChoice Human Total RNA Survey Panel, Ambion). $\Delta$Ct values were obtained using the Ct of snRNA U6 as endogenous control.

**Tissue specificity**

To calculate the tissue specificity of each miRNA, respective to the panel of analyzed samples, a score was calculated as the relative entropy of each miRNA, compared to a uniform distribution across tissues:

$$TS_i = \sum_j p_{ij} \log_2 \frac{p_{ij}}{q_i}$$

where $p_{ij}$ is the ratio between the expression level of miRNA i in tissue $j$ and the sum of all expression levels across tissues and $q_i$ is the fractional amount of miRNA i assuming a uniform distribution.

**miRNA transfections**

HepG2 cells were transfected with Lipofectamine RNAiMAx (Invitrogen), according to manufacturer's instructions. miRNA mimics and controls were obtained from Qiagen and included hsa-miR-122, hsa-miR-885-5p, AllStars Negative Control (AllStars) and AllStars Hs Cell Death Control (CDC). AllStars has no homology to any known mammalian gene, while CDC is a blend of siRNAs targeting human genes that are essential for cell survival. All oligonucleotides were transfected at 100 nM concentration, except for CDC, which was used at 20 nM.

**Gene expression profiling and Ingenuity pathway analysis**

Gene expression profiling was performed on HepG2 cells with HumanHT-12 v4 Expression BeadChip arrays (Illumina) 12 and 24 hrs post transfection. Briefly, RNA was extracted and 250 ng of total RNA (RIN> 8) was reverse transcribed and then biotinylated cRNA was generated using Illumina TotalPrep RNA Amplification kit (Ambion). Hybridization, washing and staining were performed according to the standard Illumina protocol. Data were processed with GenomeStudio V2009.1 arrays were quantile normalized, with no background subtraction, and average signals were calculated on probe-level data for genes whose detection p-value was lower than 0.001 in at least one of the groups considered.
miRNA targets were predicted using TargetScanHuman, release 5.2, considering both conserved and poorly conserved target sites. For each miRNA, the targets that were expressed in the analyzed dataset were associated with a ratio between miR-885-5p and AllStars transfected cells or between miR-122 and AllStars transfected cells. Transcripts with a ratio lower or equal to 0.7 were considered as anti-correlated to the transfected miRNA. These transcripts were used for pathway analysis on IPA (Ingenuity Pathway Analysis, http://www.ingenuity.com). The relative enrichment of biological functions and canonical pathways was determined and scored with the Fisher exact test. The final networks were created considering only experimentally-observed interactions.

**Apoptosis assays**

Apoptosis was assessed by FACS analysis based on the extent of phosphatidylserine externalization 24 and 48 hrs post transfection, using FITC Annexin V Detection kit (BD Pharmingen). DNA fragmentation was also evaluated by FACS using Propidium Iodide staining of ethanol-fixed cells 96h after transfection. A FACS Canto II and FlowJo software were used for data acquisition and analysis.

**Results**

**Profiling of serum miRNAs**

The authors generated serum miRNA profiles from healthy donors (HD) or HCV-infected patients affected by either chronic hepatitis (CH), liver cirrhosis (LC) or hepatocellular carcinoma (HCC) (Discovery Group, see Table 1). miRNAs were filtered on the basis of their detectability (Ct < 40) by TaqMan Low Density Arrays, considering as expressed those miRNAs being detected in at least two third of samples per group. Overall, 91 miRNAs resulted simultaneously detected in all groups, while 138, 101, 124 and 130 miRNAs were detected in HD, CH, LC and HCC groups, respectively.

**Reference miRNAs for adequate data normalization**

The authors initially investigated if suitable reference miRNA could be identified for data normalization. The ultimate goal was ensuring an adequate reduction of the experimentally-induced variability. The process workflow and the explanation of the variables are shown in Figure 8. Firstly, the authors used a synthetic miRNA (ath-miR-159a) as external control, within the RNA lysis buffer. With the expression of the miRNA levels relative to this reference, the authors could correct for differences in extraction or reverse transcription efficiencies. To further reduce experimental variability, the authors also used an endogenous control to eliminate the variability linked to different quality and quantity of the starting RNA amount. This endogenous reference was either a global parameter, such as the mean of all expressed miRNAs, or a normalization factor calculated from the levels of the reference miRNAs. The first strategy is well-established, and the latter is also reasonable because miRNAs showing a normalization performance similar to the mean expression value have been proposed as candidate normalizers[33].

Three different algorithms were used to reveal the best internal reference miRNAs (geNorm, Normfinder, CV score, Table 4).

**Table 4.** Top-rated miRNAs identified as endogenous controls.

| miRNA | geNorm | Normfinder | CV | Distance | Average Ct |
|---|---|---|---|---|---|
| hsa-miR-126 | 0.583 | 0.216 | 0.763 | 0.984 | 25.01 |
| hsa-miR-17 | 0.737 | 0.267 | 0.651 | 1.019 | 24.97 |
| hsa-miR-30c | 0.637 | 0.240 | 0.807 | 1.056 | 26.78 |
| hsa-miR-30b | 0.653 | 0.280 | 0.790 | 1.062 | 26.75 |
| Median | 0.253 | 0.321 | 0.985 | 1.067 | - |
| Geometric mean | 0.253 | 0.339 | 0.999 | 1.084 | - |
| hsa-miR-484 | 0.707 | 0.392 | 0.741 | 1.097 | 25.78 |
| hsa-miR-20a | 0.779 | 0.375 | 0.686 | 1.104 | 25.64 |
| hsa-miR-106a | 0.883 | 0.270 | 0.666 | 1.139 | 25.09 |

The final ranking was obtained summarizing the three scores (Figure 1A and Figure 9). There is a good agreement between the three methods, suggesting that in principle an accurate selection of low-scored miRNAs can be used to calculate the NF. Accordingly, global parameters (mainly the median or the geometric mean RQ value)were top-ranked in the same analysis. The authors selected the three miRNAs with the lowest average Ct value, namely miR-17, miR-126 and miR-484, and the authors used either the geometric mean of these three miRNAs, or the global arithmetic/geometric mean of all expressed miRNAs per sample, to obtain the miRNA Normalized Relative Quantities (NRQs). To test the hypothesis that a combination of reference miRNAs may be suitable in reducing data variation, the authors evaluated the cumulative distribution of the coefficient of variation (CV) of NRQ and not-normalized data (Figure 1B). Overall, the lowest CV values were observed after normalization with the identified reference miRNAs, indicating a substantial elimination of experimentally-induced variability. The reduction toward lower CV values was highly significant for the 50% most variable miRNAs, ($p = 0.008$, paired t-test) but not for the 50% least variable ($p = 0.657$). The same observation occurred with the other two normalization designs. This pattern of reduction in data variability has been proposed to reduce the number of false negatives[33].

Candidate normalizers were further evaluated by Principal Component Analysis (PCA), which represented an independent approach to classify miRNAs on the basis of their expression profile. Analysis of PC1 vs PC2 from autoscaled data is helpful in identifying and evaluating reference miRNAs[34].Interestingly, the selected internal reference miRNAs were clustered together, showing similar stability (Figure 1C). On the contrary, more variable miRNAs that were identified as differentially represented (see next section) were located away from the cluster of stable elements. In summary, the authors selected miR-17, mir-126 and miR-484 as internal-reference miRNAs on the basis of a combination of *in silico* scoring methods and the authors tested their ability to effectively reduce data variation, before proceeding to differential testing.

**Circulating miRNAs are differentially-represented in the serum of HCV-infected patients**

After normalization, the 91 miRNAs being consistently detected were tested for differential expression (ANOVA, p<0.05 with Bonferroni correction) and then hierarchical clustering was performed on samples and miRNAs. The list of differentially-represented miRNAs (Table 5) was obtained using data normalized with a global parameter, namely the mean of RQs of all expressed miRNAs, or alternatively using the selected reference miRNAs (miR-484, miR-17 and miR-126).

**Table 5.** miRNAs differentially represented among patients.

| miRNA | Sequence | p-value* (global mean as reference) | p-value* (miRNAs as reference) |
|---|---|---|---|
| hsa-miR-122 | UGGAGUGUGACAAUGGUGUUUG SEQ ID NO. 1 | 2.35E-03 | 6.38E-06 |
| hsa-miR-135a* | UAUAGGGAUUGGAGCCGUGGCG SEQ ID NO. 2 | 1.77E-04 | 1.48E-03 |
| hsa-miR-138-1* | GCUACUUCACAACACCAGGGCC SEQ ID NO. 3 | 1.59E-03 | 8.93E-03 |
| hsa-miR-140-5p | CAGUGGUUUUACCCUAUGGUAG SEQ ID NO. 4 | 3.35E-03 | 1.83E-03 |
| hsa-miR-16 | UAGCAGCACGUAAAUAUUGGCG SEQ ID NO. 5 | 3.79E-05 | 1.18E-07 |
| hsa-miR-188-5p | CAUCCCUUGCAUGGUGGAGGG SEQ ID NO. 6 | 2.80E-07 | 8.10E-04 |
| hsa-miR-195 | UAGCAGCACAGAAAUAUUGGC SEQ ID NO. 7 | 4.55E-05 | 7.75E-06 |
| hsa-miR-197 | UUCACCACCUUCUCCACCCAGC SEQ ID NO. 8 | 2.22E-02 | n.s.[†] |
| hsa-miR-19a | UGUGCAAAUCUAUGCAAAACUGA SEQ ID NO. 9 | 1.48E-03 | 8.20E-04 |
| hsa-miR-19b | UGUGCAAAUCCAUGCAAAACUGA SEQ ID NO. 10 | 7.02E-03 | 2.84E-05 |
| hsa-miR-19b-1* | AGUUUUGCAGGUUUGCAUCCAGC SEQ ID NO. 11 | 1.62E-02 | 7.17E-03 |
| hsa-miR-21 | UAGCUUAUCAGACUGAUGUUGA SEQ ID NO. 12 | n.s.[†] | 1.03E-05 |
| hsa-miR-223 | UGUCAGUUUGUCAAAUACCCCA SEQ ID NO. 13 | 2.68E-08 | 9.88E-15 |
| hsa-miR-24 | UGGCUCAGUUCAGCAGGAACAG SEQ ID NO. 14 | 1.50E-02 | 7.33E-05 |
| hsa-miR-30c | UGUAAACAUCCUACACUCUCAGC SEQ ID NO. 15 | n.s.[†] | 5.51E-05 |
| hsa-miR-323-3p | CACAUUACACGGUCGACCUCU SEQ ID NO. 16 | 9.25E-03 | 1.57E-03 |
| hsa-miR-335 | UCAAGAGCAAUAACGAAAAAUGU SEQ ID NO. 17 | n.s.[†] | 3.74E-02 |

(continued)

| miRNA | Sequence | p-value* (global mean as reference) | p-value* (miRNAs as reference) |
|---|---|---|---|
| hsa-miR-378 | ACUGGACUUGGAGUCAGAAGG SEQ ID NO. 18 | 2.90E-03 | 5.01E-03 |
| hsa-miR-483-5p | AAGACGGGAGGAAAGAAGGGAG SEQ ID NO. 19 | 4.51E-04 | 1.36E-06 |
| hsa-miR-509-3p | UGAUUGGUACGUCUGUGGGUAG SEQ ID NO. 20 | 1.21E-02 | 1.28E-03 |
| hsa-miR-571 | UGAGUUGGCCAUCUGAGUGAG SEQ ID NO. 21 | 6.56E-03 | 3.23E-03 |
| hsa-miR-610 | UGAGCUAAAUGUGUGCUGGGA SEQ ID NO. 22 | 3.32E-08 | 1.37E-06 |
| hsa-miR-645 | UCUAGGCUGGUACUGCUGA SEQ ID NO. 23 | 1.19E-05 | 1.03E-05 |
| hsa-miR-760 | CGGCUCUGGGUCUGUGGGGA SEQ ID NO. 24 | 9.91E-06 | 1.47E-04 |
| hsa-miR-885-5p | UCCAUUACACUACCCUGCCUCU SEQ ID NO. 25 | 2.20E-02 | 6.49E-05 |
| * after Bonferroni correction, † n.s. = not significant ($p > 0.05$) | | | |

Remarkably, 21 miRNAs were identical between the two strategies and also the corresponding clustered heat maps appeared comparable (Figure 2 and Table 2). Using these differentially-represented miRNAs for unsupervised hierarchical clustering, the two higher-order clusters separated a first group enriched in HD and CH patients and a second group mostly populated by patients with LC and HCC (Figure 2). A similar analysis was performed comparing only HD and patients with advanced liver diseases, namely LC and HCC. The resulting signature discriminated between the two classes and also improved the unsupervised clustering (Figure 10). In summary, the authors identified differentially-represented miRNAs circulating in the serum of HCV-infected patients. These miRNAs carry valuable information for a genuine classification of the samples because a specific microRNA serum signature is able to describe the disease status of infected patients.

**Circulating miRNAs miR-122, miR-483-5p and miR-885-5p reflect disease progression**

Interestingly, miR-122, which was rated among the 21 differentially-represented miRNAs, has been consistently associated with hepato-carcinogenesis. It is the most expressed miRNA in normal liver and its level has been shown to vary in HCC [23-28]. Importantly, miR-122 is specifically required for HCV replication and infectious virus production [5,35]. Based on this knowledge, the authors performed a template-matching search using the profile of miR-122 to specifically fish for miRNAs whose profile was correlated to that of miR-122 itself. Using a Pearson R cutoff value of 0.6, miR-21, miR-483-5p, miR-610 and miR-885-5p were identified and selected for further characterization and classification. These five miRNAs were also grouped together by PCA and the first two principal components classified them among the most variable (Figure 1C). Their representation was low in healthy individuals but increased progressively in patients with CH, LC and HCC, mirroring the progression of the disease (Figure 3). Hence, to assess whether these five miRNAs could correctly classify the samples on the basis of their profile, the authors used the PAM and SVM algorithms. Using PAM, the best performance was obtained specifying two groups and 92% of the samples were correctly classified as healthy donors or HCV-infected patients (Table 6). With the SVM algorithm, the classification generally improved (Table 6).

**Table 6.** Performance of PAM and SVM classification

| Method | Groups | Correct assignments (%) | Accuracy (%)* |
|---|---|---|---|
| PAM | 2 (HD, HCV) | 92 | 92 |
|  | 3 (HD, CH, LC-HCC) | 82 | 80 |
|  | 4 (HD, CH, LC, HCC) | 62 | 50 |

(continued)

| Method | Groups | Correct assignments (%) | Accuracy (%)* |
|--------|--------|------------------------|---------------|
| SVM | 2 (HD, HCV) | 98 | 96 |
| | 3 (HD, CH, LC-HCC) | 94 | 78 |
| | 4 (HD, CH, LC, HCC) | 82 | 56 |

* after ten-fold cross-validation

Indeed, 98% of the samples were correctly assigned to the true class performing a two group classification (HD vs. HCV-infected), Using a ten-fold cross validation the authors were able to estimate the accuracy of the model, showing that the accuracy values ranged from a maximum of 96% using a two-group classification to a minimum of 56% using a four-group classification.

In order to check for the specificity of this association, the authors included a group of sera from patients with Crohn's disease (CD) that were collected during the active phase of gut inflammation, because this condition represents another form of immune-mediated disease where the target organ is different than the liver. Three out of five miRNAs (miR-122, miR-885-5p and miR-483-5p) were specifically and consistently increased in all the three groups of HCV-infected patients (CH, LC and HCC) compared with CD (Figure 3). Furthermore, they showed the widest fold-change, reaching a 10-fold difference compared to controls. Conversely, miR-21 and miR-610 showed a much smaller fold change with respect to CD, suggesting that their increased representation in serum could not be specific for liver disease.

Together, these data show that circulating miRNAs can be used for proper classification of patients, especially to distinguish between HD and patients with advanced liver diseases (LC and HCC). Furthermore, circulating miRNAs miR-122, miR-483-5p and miR-885-5p are highly increased in the serum of patients with HCV infection and their levels are specific for liver inflammation.

## Cellular origin of disease-enriched miRNAs

The current model proposes that miRNA are present in blood circulation either in a membrane-bound compartment, such as inside exosomes, or in a vesicle-free form, mainly associated to protein complexes [36]. The authors investigated a preferential enrichment in one of the two compartments of disease-enriched miRNAs identified above. Therefore, the authors evaluated the representation of the whole miRNome in matched samples of whole serum and exosomes purified by differential centrifugation using fresh serum specimens from four healthy donors.The authors detected 183 miRNAs in total serum and 85 miRNAs in the exosome preparations, in at least three out of four samples with a Ct<35.The authors then selected 28 miRNAs that were well represented in total serum but not detectable in purified exosomes (Ct ≤ 30 in at least three out of four serum samples and a Ct ≥ 35 in in at least three out of four exosome samples). Notably, miR-122, miR-483-5p and miR-885-5p resulted among these exosome-depleted miRNAs, highlighting that they are not preferentially partitioned in vesicles (Figure 4A). Conversely, miR-21 was identified in both total serum and exosomes preparations.

To gain further insight into the cellular origin of disease-enriched serum miRNAs, a panel of RNA from different human tissues including hepatoma cell lines was tested by quantitative RT-PCR (Figure 4B). miR-122 was found to be highly abundant in liver tissue as expected [37] and similarly miR-885-5p was mostly expressed in liver and to a lesser extent in brain and skeletal muscle, suggesting that the liver may be the main originating tissue responsible for the observed increase of circulating miRNA levels in sera of HCV patients. Besides, miR-483-5p was expressed in all tissues at a comparable level, including liver. A quantitative analysis was performed calculating the Tissue Specificity score, as the relative entropy for each miRNA, comparing the experimentally observed expression in each tissue with respect to an expected expression resulting from a uniform distribution across tissues. miR-122 and miR-885-5p had the highest information content indicating their high tissue specificity (Figure 4C).

Finally, considering their specific liver expression, miR-122 and miR-885-5p were selected for further analysis. To characterize the accumulation of liver-related miRNAs in the blood circulation, the authors profiled a panel of serum samples from 67 patients with HCV infection (Validation Group I, see Supplementary table 2) and considered two common parameters that are measured in the serum during chronic infection: the levels of alanine aminotransferase (ALT) activity, a marker commonly used for the assessment of liver damage, and the viral load, reflecting the extent of liver infection. To specifically quantify miR-122 and miR-885-5p in serum ,the authors calculated the RQs using the above selected reference miRNAs as normalizers. Both miR-122 and miR-885-5p serum concentration positively correlated with ALT activity ($p < 10^{-9}$, Pearson R>0.7, Figure 5).However, the authors did not detect a correlation between the viral load and the levels of the miRNAs in the serum of infected patients.

All together, these results are consistent with a release from the liverof miR-122 and miR-885-5p in a non-vesicular form, as a consequence of liver damage following inflammation, instead of a pure release from infected hepatocytes, as a consequence of viral replication.

**Circulating miRNAs miR-122 and miR-885-5p predict HCC**

A major question is whether circulating miRNAs could discriminate between patients with or without HCC. Therefore, in order to eliminate the odds that subtle differences could be masked by inter-individual variability, the authors inspected the expression of these miRNAs in longitudinal samples collected from the same patient before and after the diagnosis of HCC (Validation Group II, See Supplementary Table 3).The authors included also cases with etiology different than HCV (including HBV), in order to highlight potential differences. Overall, after HCC development, the authors observed a highly significant decrease in serum levels of miR-122 and miR-885-5p (p=7.66E-03 and p=1.22E-04 respectively),occurring with a decrease in ALT (p=0.0149) but not AFP levels (Figure 6A). However, upon stratification of HCV vs. non-HCV etiology (Figure 11),the authors observed that the difference in ALT levels remained significant only for patients with non-HCV etiology (p=0.0325).Importantly, mir-122 or miR-885-5p levels did not correlated with ALT activity, in this group of longitudinal samples, even after stratification. A bidimensional representation using the miRNA-ALT variables showed that it is possible to identify clusters that reflect disease etiology (Figure 6B) and differ in the relative serum levels of miR-122 and ALT. Using ROC curve analysis the authors showed that miR-122, miR-885-5p or a combination of both have similar or even better performance compared to ALT and AFP to predict the HCC class (Figure 6C). Using the combination of miR-122 and miR-885-5p the AUC (area under the curve) was 0.933

**What is the biological role of and miR-885-5p within the liver?**

Considering that serum levels of miR-885-5p were always highly correlated in in or analyses to those of miR-122, a key miRNA in liver biology, the authors asked whether it could also be possible to envisage a functional role for miR-885-5p in liver and its possible involvement in the progression of chronic disease toward cancer. The authors performed gene-expression analysis of hepatoma cell lines transiently transfected with miRNA mimics, in order to look for responsive targets responsive to augmented miR-885-5p expression. The authors compared miR-885-5p transfected HepG2 cells *versus* negative control-transfected cells combining the prediction of miR-885-5p targets (using Targetscan 5.2) with their expression values. The expressed miR-885-5p predicted targets whose expression was reduced at least 0.7 times in miR-885-5p transfected cells were selected for mapping biomolecular networks based on known pathways, gene ontology and interactions, using Ingenuity Pathway Analysis. The authors identified 11 transcripts negatively correlated to the expression of miR-885-p and a resulting network was generated (Figure 12). Interestingly, the network included SET gene as a candidate key target of miR-885-5p. The product of this gene is part of the SET complex, which plays a critical role in DNA repair and damage, oxidative stress response and caspase-independent cell death, morphologically indistinguishable from apoptosis [38,39]. Moreover, TP53 and MYC genes also figured in the interaction network, further highlighting the potentially critical activity of miR-885-5p as a positive regulator of cell death. In the authors' experiments, SET transcript was progressively reduced, with a maximum decrease of 45% 24 hrs after miR-885-5p transfection, negatively correlating with miR-885-5p (Figure 7A). In order to validate this approach, the same analysis was performed after overexpression of miR-122. In this case, 55 transcripts were identified using the same criteria, including previously known validated responsive miR-122 targets, such as ALDOA [40] and SLC7A1 (CAT-1) [37]. The corresponding networks were enriched in established biological functions of miR-122 target genes within the liver, such as amino acid metabolism, cancer and cell death (data not shown).

Next, the authors decided to experimentally validate the involvement of miR-885-5p in nuclear damage and cell death. To do so, the authors measured both the percentage of sub-G1 cells 96 hrs after miR-885-5p transfection and the percentage of cells stained as Annexin-V positive and Propidium Iodide negative, 24 and 48 hrs after miR-885-5p transfection in HepG2 cell line. The authors observed an increase of cells showing phosphatidyl serine externalization, a common hallmark of apoptotic cells (Figure 7C), and also a remarkable increase of DNA fragmentation, as revealed by the presence of sub-G1 peaks (Figure 7B). In conclusion, miR-885-5p is both a biomarker of HCV-associated liver diseases and likely an interesting player in hepatocyte biology, as revealed by the integration of *in silico* and experimental results, showing it as a modulator of cell death.

**Discussion**

In this work the authors described the profiling of miRNAs in the serum of patients with chronic HCV infection. The authors observed that the levels of liver-specific miRNAs are associated with the disease status. Specifically, mir-122 and miR-885-5p were found to be over-represented in the sera of patients with chronic HCV infection, with increasing concentration as the disease progressed to advanced phases, such as LC and HCC. Moreover, this increased representation was specific to chronic liver inflammation. These miRNAs also showed high sensitivity and specificity in predicting HCC class in a group of longitudinal observations. The authors' work included a solid normalization approach and finally led to the proposal of a novel role for miR-885-5p in liver biology.

Over the last few years, accumulating evidence supported the use of miRNAs circulating in blood as non-invasive biomarkers of disease conditions, particularly in cancer [11,13,12]. The authors were particularly interested in finding a miRNA signature with diagnostic potential in the setting of chronic HCV infection, considering that up to 20% of chronically infected patients may develop severe complications including cirrhosis, liver failure or hepatocellular carcinoma. While several publications have described a correlation between serum levels of miR-122 and hepatic damage due to hepatitis or different types of liver injury in both mice and man[41-44],only one study has previously associated serum levels of miR-885-5p to advanced liver disease, i.e. liver cirrhosis and HCC in a cohort including mainly cases with HBV etiology [45]. However, the authors did not report any association with transaminase activity, although they observe a specific increase in the serum of patients with liver pathologies. The major differences with the authors' analysis include a different study design, because the authors focused exclusively on chronic HCV infection, and a different normalization of the data, because they used the U6 small nuclear RNA that in the authors' analysis resulted having low stability and thus was not selected as reference RNA.

The authors strongly believe that an important requirement for the identification of candidate biomarkers is a solid normalization strategy to account for inter-individual or intergroup variability. Indeed, there is growing evidence that the small RNAs RNU44, RNU48 and U6, routinely used as reference RNAs in cellular studies, are highly variable in serum and their use is now considered highly controversial [11], thus underlying the importance of finding suitable stable miRNAs that are stably detectable in human serum. In order to identify normalizer miRNAs in the authors' dataset, the authors exploited multiple bioinformatic approaches (geNorm, Normfinder, PCA), previously acknowledged as valuable tools for the discovery of stable reference transcripts. Usually, the authors observed that the yield of RNA from small volume plasma or serum samples (i.e., 100-400 $\mu$l) is below the limit of accurate quantification [46]. The authors evaluated serum miRNA expression starting from clinical samples, in a retrospective manner, using limited amount (70 $\mu$l) of frozen serum. Therefore, a normalization based on a fixed amount of starting RNA was not feasible and instead the authors used a fixed volume of starting material and developed a solid experimental workflow to provide a robust normalization strategy, with the goal of minimizing data variation. The effect of a well-designed normalization process is highlighting true biological changes and eliminating, or at least reducing, the variability introduced during the whole experimental process. For array-based approaches, a normalization strategy based on the mean value of miRNA expression has been proposed for qPCR data [33]. This method is only applicable when high-throughput miRNA profiling is performed. The authors successfully obtained candidate reference miRNAs that were valuable in reducing variability, as shown by the cumulative distribution of CV values of normalized data. Obtaining a number of solid normalizers is extremely advantageous, considering the prerequisite of reducing the number of reference miRNAs and increasing the throughput and the feasibility of single or focused qPCR assays. In fact, a large-scale prospective study would be extremely advantageous to investigate the role of circulating miRNAs in the progression from liver cirrhosis to HCC, taking advantage of the results of the present study of both stable and differentially-represented miRNAs in chronic HCV infection.

In the authors' analyses, miR-122 and miR-885-5pshowed a common liver origin. This observation does not rule out the possibility that their increase in serum during the pathogenetic process of the liver in HCV natural history could be mainly immune-mediated. Indeed, liver infiltrating lymphocytes are observed during chronic infection, which is associated with portal inflammation, periportal necrosis, fibrosis and often steatosis. Furthermore, lymphocytes constitute normally 5-10% of the liver of a healthy individual [47]. Hence, to assess whether the miRNA signatures were specific for chronic hepatitis C or they could be more generally exploited as a hallmark of inflammation, the authors checked for the specificity using a group of sera from patients with CD. Actually, CD is a form of inflammatory bowel disease characterized by a marked infiltration into the intestinal lamina propria of immune cells of the innate and adaptive compartment, mediated by an overactive Th17 and Th1 pro-inflammatory cytokine response [48]. Hence, this condition represented an ideal control to check for the presence of circulating miRNAs in another disease characterized by immune-mediated tissue injury, similarly to chronic HCV infection, when the target organ is different than the liver. The authors' observation that miR-122, miR-483-5p and miR-885-5p were increased in all groups of patients with liver diseases not only compared to healthy controls but also to patients with CD, further supporting the specificity of the association. Although miR-21 concentration was also increased in LC and HCC groups, it is well known that its circulating levels are also increased in a variety of other diseases, including many types of cancers [11-13], in agreement with its broad expression across different tissues. Thus, although the determination of serum miRNA-21 level can be useful to monitor the presence of advanced liver complications, the authors did not selected this miRNA for further analysis.

The changes in the concentration of specific miRNAs in the serum may reflect variations both in their expression within the originating tissue and/or release within the blood circulation. The latter mechanism accounts for a combination of passive release, mainly related to cell death, and controlled release, mainly through vesicles containing miRNAs[49-51]. A change in the release from the originating tissue is a reasonable possibility during a disease characterized by chronic and progressive injury, as observed in the liver during HCV infection. Hence, the authors believe that an increased miRNA release from the liver tissue of patients with HCV infection is responsible for the reported differences in the levels of miR-122 and miR-885-5p. Additionally, the authors observed that miR-122 and miR-885-5p were always detected in the whole serum but never in the exosomes isolated by differential centrifugation from serum specimens of healthy donors. The preferential exclusion of these miRNAs from the exosome compartment may be suggestive of a passive release from the liver, after necrosis or apoptosis and accumulation within cell debris, protein complexes or apoptotic bodies. Consistently with this hypothesis, the authors evidenced that miR-122 and miR-885-5p serum levels are strongly associated with ALT activity, the most widely used biochemical marker for measuring liver injury. This finding is consistent with previous observations in mice showing a correlation between serum levels of miR-122 and hepatic damage after drug-induced injury [41]. Notably, the mouse does not have a homolog for miR-885-5p. Recently, other findings confirmed that circulating miR-122 levels are correlated to liver injury also in man. [42,43].

The authors further investigated whether miR-122 and miR-885-5p could be exploited as biomarker of HCC occurrence. In the authors' discovery group, their level did not significantly changes between LC and HCC classes. However, when new samples were analyzed longitudinally before and after the diagnosis of HCC, the authors were able to show a decrease after the occurrence of HCC. Moreover, in this Validation Group II, miR-122 or miR-885-5p did not correlate with ALT activity, in contrast to what observed with the Validation Group I. The difference may be explained considering the different characteristics of the respective patients. The Validation Group II is composed of paired samples collected before and after the occurrence of HCC, while the Validation Group I is composed by independent patients infected with HCV and collected during different phase of the disease, including acute or chronic hepatitis, liver cirrhosis and HCC.miR-122 is the most expressed microRNA in normal liver, and it is required for HCV replication and infectious virus production [5,35]. Its role in tumorigenesis has been long investigated. Indeed, miR-122 levels have been shown to decrease in HCC [23-25,27,28], although one study reported an up-regulation in HCV-associated HCC [26]. Therefore, the authors speculate that during HCC establishment, the circulating levels of deregulated miRNAs may be fine-tuned as a result of increased release after liver damage at one side and decreased cellular expression at the other. This could explain why the authors observed a decrease in circulating miRNA levels only with paired design, when the inter-individual variation is eliminated.

miR-122 is considered a tumor-suppressor for its role in regulating apoptosis, response to chemotherapeutics and cell migration and invasiveness [29,52,53].In the authors' analyses, miR-122 and miR-885-5p were closely linked: they exhibited similar high variability in the analysis of normalizers, were clustered together using both hierarchical clustering and PCA and finally showed a common liver origin. These considerations prompted the authors to investigate the role of miR-885-5p in liver biology, in search of potential overlapping functions with miR-122.Notably,miR-885-5p has been recently shown to induce p53 activation and cell death in neuroblastoma cells [54]. In the present work, miR-885-5p revealed interesting potential functions in the liver that surely deserves further investigation. In fact, bioinformatic pathway analysis of modulated transcripts revealed a network populated with genes involved in cell death and DNA recombination and repair. Indeed, the authors' experimental data support the hypothesis that miR-885-5p is capable of inducting apoptosis in hepatoma cell lines. This process is likely to be mediated by the down-regulation of the SET transcript operated by miR-885-5p. The SET nuclear oncogene (also known as IGAAD or IPP2A2) is a member of the SET complex and is an inhibitor of the nuclease NM23-H1. Moreover, SET is an inhibitor of the tumor-suppressor PP2A. The SET complex translocates from the ER into the nucleus where it mediates the nuclear damage observed in Granzyme A mediated cell death [39]. This phenomenon is morphologically indistinguishable from apoptosis, is caspase-independent and is mainly observed after release of cytotoxic granules by cytotoxic T lymphocytes and natural killer cells. However, one study reported the cleavage of SET by the AEP protease in neurons after acidification independently of immune cells [55], opening the possibility that this complex can be involved in a wider regulation of cell death. Importantly, SET protein has been recently found to be up-regulated in HCC [56], providing a possible mechanistic involvement of this protein, and hence of its regulating miR-885-5p in liver carcinogenesis.

In conclusion, the authors established the promising role of circulating miRNAs as biomarkers for liver diseases. The authors demonstrated that miR-122 and miR-885-5p specific serum increase is a hallmark of chronic liver inflammation and the authors provided initial evidences for a direct involvement of miR-885-5p in liver biology and a possible involvement in the molecular processes occurring during cellular neoplastic transformation. The authors also showed that miR-122 and miR-885 showed optimal performance in classifying paired samples before and after the occurrence of HCC. The authors' work represents a solid discovery phase, designed to screen for potential miRNAs to be further validated. In a second-phase larger validation study, the authors will assess the strength of the reported association, the sensitivity and specificity of circulating miRNAs as biomarker of HCV-associated liver diseases.

**Bibliography**

1. Bartel DP Cell 2009, 136:215-233

2. Guo H, et al., Nature 2010, 466:835-840

3. Volinia S, et al., Proc Natl Acad Sci USA 2006, 103:2257

4. O'connell RM, et al., Nature Reviews Immunology 2010, 10:111-122

5. Jopling CL, et al., Science 2005, 309:1577-1581

6. Liang Y, et al., BMC Genomics 2007, 8:16610.1186/1471-2164-8-166.

7. Lu J, et al., Nature 2005, 435:834-838

8. Chen X, et al., Cell Res 2008, 18:997-1006

9. Mitchell PS, et al., Proc Natl Acad Sci USA 2008, 105:10513-10518

10. Gilad S, et al., PLoS ONE 2008, 3:e314810.1371

11. Reid G, et al., Critical reviews in oncology/hematology 2011, 80:193-208

12. Kosaka N, et al., Cancer Science 2010, 101:2087-2092

13. Etheridge A, et al., Mutat Res 2011, 717:85-90

14. Lauer GM, Walker BD, N Engl J Med 2001, 345:41-52

15. Manns MP, et al., The Lancet 2001, 358:958-965

16. Fried MW, et al., N Engl J Med 2002, 347:975-982

17. Kim WR, et al., Hepatology 2008, 47:1363-1370

18. Nathwani RA, et al., Hepatology 2005, 41:380-382

19. Korones DN, et al., Am. J. Hematol. 2001, 66:46-48

20. Bruix J, et al., J Hepatol 2001, 35:421-430.

21. Daniele B, et al., Gastroenterology 2004, 127:S108-12.

22. Gebo KA, et al., Hepatology 2002, 36:S84-92

23. Kutay H, et al., J Cell Biochem 2006, 99:671-678

24. Gramantieri L, et al., Cancer Research 2007, 67:6092-6099.

25. Meng F, et al., Gastroenterology 2007, 133:647-658.

26. Varnholt H, et al., Hepatology 2008, 47:1223-1232

27. Ura S, et al., Hepatology 2009, 49:1098-1112

28. Chen X-M, World J Gastroenterol 2009, 15:1665-1672.

29. Lewis AP, Jopling CL: Biochem. Soc. Trans 2010, 38:1553-1557

30. Vandesompele J, Genome Biol 2002, 3:RESEARCH0034.

31. Andersen CL, et al., Cancer Research 2004, 64:5245-5250.

32. Théry C, et al., Curr Protoc Cell Biol 2006, Chapter 3:Unit 3.2210.1002

33. Mestdagh P, et al., Genome Biol 2009, 10:R6410.1186/gb-2009-10-6-r64.

34. Vandesompele J, Kubista M, Pfaffl MW: Reference gene validation software for improved normalization. 2008, :1-18.

35. Randall G, et al., Proc Natl Acad Sci USA 2007, 104:12884-12889

36. Arroyo JD, et al., Proc Natl Acad Sci USA 2011, 108:5003-5008

37. Chang J, et al., RNA Biol 2004, 1:106-113.

38. Beresford PJ, Journal of Biological Chemistry 2001, 276:43285-43293

39. Chowdhury D, Lieberman J, Annu Rev Immunol 2008, 26:389-420

40. Krützfeldt J, et al., Nature 2005, 438:685-689.

41. Wang K, et al., Proc Natl Acad Sci USA 2009, 106:4402-4407

42. Zhang Y, et al., Clinical Chemistry 2010, 10.1373/clinchem.2010.147850.

43. Starkey Lewis PJ, et al., Hepatology 2011, 54:1767-1776

44. Cermelli S, et al., PLoS ONE 2011, 6:e2393710.1371

45. Gui J, et al., Clin. Sci. 2011,120:183-193

46. Kroh EM, et al., Methods 2010, 50:298-301

47. Racanelli V, et al., Hepatology 2006, 43:S54-S62

48. Abraham C, Cho JH. N Engl J Med 2009, 361:2066-2078

49. Valadi H, et al., Nat Cell Biol 2007, 9:654-659

50. Gibbings DJ, et al., Nature 2009, 11:1143-1149

51. Gibbings D, et al., Trends Cell Biol 2010, 20:491-501

52. Fornari F, et al., Cancer Research 2009, 69:5761-5767

53. Coulouarn C, et al., Oncogene 2009, 28:3526-3536

54. Afanasyeva EA, et al., Cell Death and Differentiation 2011, 18:974-984

55. Liu Z, et al., Molecular Cell 2008, 29:665-678

56. Zeng R, et al., J Proteome Res 2011, :111114185354007

**Claims**

1.  A method for the diagnosis and/or prognosis of a liver disease in a subject or to identify a subject at risk to develop a liver disease comprising the following steps:

    a) measuring the amount of a miR-122 and a miR-885-5pmolecule, an equivalent such as a mimic or an isomiR thereof in a biological sample isolated from the subject, and
    b) comparing the measured amount of step a) with an appropriate control amount of a miR-122 and a miR-885-5pmolecule, an equivalent such as a mimic or an isomiR thereof, wherein if the amount of the miR-122 and the mir-885-5pmolecule, an equivalent such as a mimic or an isomiR thereof in the biological sample is altered with respect to the control amount, then the subject is affected by a liver disease or is at risk of developing a liver disease.

2. The method according to claim 1 further comprising measuring the amount of a miR-483-5p molecule, an equivalent such as a mimic or an isomiR thereofand comparing said measured amount of miR-483-5p molecule, an equivalent such as a mimic or an isomiR thereofto an appropriate control amount of miR-483-5pmolecule, an equivalent such as a mimic or an isomiR thereof.

3. The method according to claim 1 or 2 wherein the liver disease is an HCV-associated liver disease.

4. The method according to any of claim 1 to 3 wherein the liver disease is selected from the group consisting of: chronic hepatitis, liver cirrhosis, hepatocellular carcinoma.

5. The method according to any one of previous claim wherein the biological sample is a blood or a serum sample.

6. A method to early diagnosing of hepatocellular carcinoma comprising:

   a) measuring the amounts of a miR-122 and a miR-885-5pmolecule, an equivalent such as a mimic or an isomiR thereof in a biological sample isolated from the subject at different times, and
   b) comparing the measured amounts of step a) wherein if the amount of the miR-122 and the mir-885-5pmolecule, an equivalent such as a mimic or an isomiR thereof in the biological sample decrease over time, then the subject has an higher risk of developing hepatocellular carcinoma.

7. A method to monitoring the progression of hepatocellular carcinoma comprising:

   a) measuring the amounts of a miR-122 and a miR-885-5pmolecule, an equivalent such as a mimic or an isomiR thereof in a biological sample isolated from the subject at different times, and
   b) comparing the measured amounts of step a).

8. A kit for the diagnosis and/or prognosis of a liver disease or to identify a subject at risk to develop a liver diseaseand/or to predict the risk of developing hepatocellular carcinoma in a subject according to any of methods of claims 1 to 7, comprising:

   - means to detect and/or measure the amount of miR-122 and miR-885-5p; and optionally
   - control means.

9. The kit according to claim 8 further comprising means to detect and/or measure the amount of miR-483-5p.

10. A modulator of miR-885-5p for use as a medicament.

11. The modulator according to claim 10 wherein the modulator is a nucleic acid molecule.

12. The modulator according to any one of claim 10or 11for use in the treatment and/or prevention of a liver disease.

13. The modulator according to claim 12 wherein the liver disease is an HCV-associated liver disease.

14. The modulator according to claim 13wherein the HCV-associated liver disease is selected from the group consisting of: chronic hepatitis, liver cirrhosis, hepatocellular carcinoma.

15. A pharmaceutical composition comprising at least one modulator as defined in any one of claim 10 to 14and excipients and/or adjuvants.

FIG. 1

A

B

FIG. 2

FIG. 3

A

Serum

| | |
|---|---|
| hsa-miR-101 | hsa-miR-339-3p |
| **hsa-miR-122** | hsa-miR-345 |
| hsa-miR-128 | hsa-miR-365 |
| hsa-miR-132 | hsa-miR-375 |
| hsa-miR-143 | hsa-miR-379 |
| hsa-miR-148a | hsa-miR-409-3p |
| hsa-miR-152 | **hsa-miR-483-5p** |
| hsa-miR-18a | hsa-miR-494 |
| hsa-miR-193a-5p | hsa-miR-532-3p |
| hsa-miR-223* | hsa-miR-539 |
| hsa-miR-28-5p | hsa-miR-660 |
| hsa-miR-30d | hsa-miR-744 |
| hsa-miR-30e | hsa-miR-768-3p |
| | hsa-miR-769-5p |
| | **hsa-miR-885-5p** |

Exosomes

B

High          Intermediate          Low

Adipose, Bladder, Brain, Cervix, Colon, Esophagus, Heart, Kidney, Liver, Huh7.5, HepG2, Lung, Ovary, Placenta, Prostate, Skeletal muscle, Small intestine, Spleen, Testes, Thymus, Thyroid, Trachea

miR-610
miR-21
miR-483-5p
miR-885-5p
miR-122

C

**Tissue Specificity**

miR-885-5p
miR-610
miR-483-5p
miR-122
miR-21

0    1    2    3    4    5

**TS**

FIG. 4

A

B

FIG. 5

A

B

C

FIG. 6

A

B

C

FIG. 7

FIG. 7

A

■ 12h SET ■ 24h SET
↦ 12h miR-885-5p ✳ 24h miR-885-5p

B

C

FIG. 7

FIG. 8

FIG. 9

FIG. 9

FIG. 9

A

B

FIG. 10

FIG. 11

FIG. 12

Europäisches
Patentamt

European
Patent Office

Office européen
des brevets

# EUROPEAN SEARCH REPORT

Application Number

EP 13 17 8245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/027332 A2 (ISTITUTO NAZ DI GENETICA MOLECOLARE INGM [IT]; PAGANI MASSIMILIANO [IT) 10 March 2011 (2011-03-10) * abstract * * page 3, line 27 - page 5, line 16; example 1; tables 1-2 * * claims 8-9 * | 1-15 | INV. C12Q1/68 |
| X | MARABITA, F.: "Single Nucleotide Polymorphisms and circulating microRNAs for monitoring HCV disease progression: an integrated approach. Tesi di dottorato, Università degli Studi di Milano-Bicocca, 2011.", Bicocca Open Archive , 29 March 2011 (2011-03-29), pages 75-110, XP002716249, Milan Retrieved from the Internet: URL:http://boa.unimib.it/bitstream/10281/2 9993/1/phd_unimib_716450.pdf [retrieved on 2013-11-13] * page 75 - page 110 * | 1-15 | |
| L | MARABITA, F.: "Single Nucleotide Polymorphisms and circulating microRNAs for monitoring HCV disease progression: an integrated approach", Bicocca Open Archive , 29 March 2011 (2011-03-29), XP002716280, Retrieved from the Internet: URL:http://hdl.handle.net/10281/29993 [retrieved on 2013-11-13] * abstract * | | |

TECHNICAL FIELDS
SEARCHED (IPC)

C12Q

-/--

The present search report has been drawn up for all claims

| Place of search | Date of completion of the search | Examiner |
|---|---|---|
| Munich | 13 November 2013 | Werner, Andreas |

EPO FORM 1503 03.82 (P04C01)

Europäisches
Patentamt

European
Patent Office

Office européen
des brevets

# EUROPEAN SEARCH REPORT

Application Number

EP 13 17 8245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | GUI, JUNHAO ET AL: "Serum microRNA characterization identifies miR - 885 - 5p as a potential marker for detecting liver pathologies", CLINICAL SCIENCE, vol. 120, no. 5, 1 March 2011 (2011-03-01), pages 183-193, XP055047582, ISSN: 0143-5221, DOI: 10.1042/CS20100297 * abstract; table 1 * * page 184, column 1, paragraph 4 - page 186, column 1, paragraph 1; figure 4 * * page 188, column 1, paragraph 3 - page 189, column 2, paragraph 1 * * page 191, column 1, paragraph 4 - page 192, column 1, paragraph 1 * | 1-15 | |
| X | CN 101 368 213 A (UNIV NANJING) 18 February 2009 (2009-02-18) * abstract; claim 2 * * examples 2-3; tables 1-2 * | 1,2,5,8, 9,15 | |
| X | GIRARD ET AL: "miR-122, a paradigm for the role of microRNAs in the liver", JOURNAL OF HEPATOLOGY, ELSEVIER, AMSTERDAM, NL, vol. 48, no. 4, 12 February 2008 (2008-02-12), pages 648-656, XP022534926, ISSN: 0168-8278, DOI: 10.1016/J.JHEP.2008.01.019 | 8,9 | **TECHNICAL FIELDS SEARCHED (IPC)** |
| A | * abstract; table 1 * | 1-7 | |

-/--

The present search report has been drawn up for all claims

| Place of search | Date of completion of the search | Examiner |
|---|---|---|
| Munich | 13 November 2013 | Werner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

**Application Number**

EP 13 17 8245

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | C COULOUARN ET AL: "Loss of miR-122 expression in liver cancer correlates with suppression of the hepatic phenotype and gain of metastatic properties", ONCOGENE, vol. 28, no. 40, 20 July 2009 (2009-07-20), pages 3526-3536, XP055087939, ISSN: 0950-9232, DOI: 10.1038/onc.2009.211 * abstract * | 1-15 | |
| A | WO 2013/095941 A1 (VALLEY HEALTH SYSTEM [US]; GANEPOLA GANEPOLA A P [US]) 27 June 2013 (2013-06-27) * page 2, line 2 - line 3; claim 1 * | 1-15 | |
| A | CN 101 921 863 A (CHINA PLA GENERAL HOSPITAL) 22 December 2010 (2010-12-22) * the whole document * | 1-15 | |
| A | CN 102 888 453 A (CHINESE PLA GENERAL HOSPITAL) 23 January 2013 (2013-01-23) * the whole document * | 1-15 | **TECHNICAL FIELDS SEARCHED (IPC)** |
| A | XUE ZHAO ET AL: "The role and clinical implications of microRNAs in hepatocellular carcinoma", SCIENCE CHINA LIFE SCIENCES, SP SCIENCE CHINA PRESS, HEIDELBERG, vol. 55, no. 10, 31 October 2012 (2012-10-31), pages 906-919, XP035131479, ISSN: 1869-1889, DOI: 10.1007/S11427-012-4384-X * abstract * * page 908, column 1, paragraph 3 - column 2, paragraph 1 * * page 912, column 1, paragraph 2 - paragraph 3 * | 1-15 | |

The present search report has been drawn up for all claims

| Place of search | Date of completion of the search | Examiner |
|---|---|---|
| Munich | 13 November 2013 | Werner, Andreas |

EPO FORM 1503 03.82 (P04C01)

**ANNEX TO THE EUROPEAN SEARCH REPORT
ON EUROPEAN PATENT APPLICATION NO.**

EP 13 17 8245

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2013

| Patent document cited in search report | | Publication date | Patent family member(s) | | Publication date |
|---|---|---|---|---|---|
| WO 2011027332 | A2 | 10-03-2011 | AU | 2010290828 A1 | 05-04-2012 |
| | | | CA | 2773411 A1 | 10-03-2011 |
| | | | EP | 2475785 A2 | 18-07-2012 |
| | | | EP | 2657354 A1 | 30-10-2013 |
| | | | US | 2012238617 A1 | 20-09-2012 |
| | | | WO | 2011027332 A2 | 10-03-2011 |
| CN 101368213 | A | 18-02-2009 | CN | 101368213 A | 18-02-2009 |
| | | | CN | 102016037 A | 13-04-2011 |
| | | | WO | 2010043114 A1 | 22-04-2010 |
| WO 2013095941 | A1 | 27-06-2013 | NONE | | |
| CN 101921863 | A | 22-12-2010 | NONE | | |
| CN 102888453 | A | 23-01-2013 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200810558 A

### Non-patent literature cited in the description

- **BARTEL DP.** *Cell,* 2009, vol. 136, 215-233
- **GUO H et al.** *Nature,* 2010, vol. 466, 835-840
- **VOLINIA S et al.** *Proc Natl Acad Sci USA,* 2006, vol. 103, 2257
- **O'CONNELL RM et al.** *Nature Reviews Immunology,* 2010, vol. 10, 111-122
- **JOPLING CL et al.** *Science,* 2005, vol. 309, 1577-1581
- **LIANG Y et al.** *BMC Genomics,* 2007, vol. 8, 16610.1186, , 1471-2164, 8-166
- **LU J et al.** *Nature,* 2005, vol. 435, 834-838
- **CHEN X et al.** *Cell Res,* 2008, vol. 18, 997-1006
- **MITCHELL PS et al.** *Proc Natl Acad Sci USA,* 2008, vol. 105, 10513-10518
- **GILAD S et al.** *PLoS ONE,* 2008, vol. 3, 314810-1371
- **REID G et al.** *Critical reviews in oncology/hematology,* 2011, vol. 80, 193-208
- **KOSAKA N et al.** *Cancer Science,* 2010, vol. 101, 2087-2092
- **ETHERIDGE A et al.** *Mutat Res,* 2011, vol. 717, 85-90
- **LAUER GM ; WALKER BD.** *N Engl J Med,* 2001, vol. 345, 41-52
- **MANNS MP et al.** *The Lancet,* 2001, vol. 358, 958-965
- **FRIED MW et al.** *N Engl J Med,* 2002, vol. 347, 975-982
- **KIM WR et al.** *Hepatology,* 2008, vol. 47, 1363-1370
- **NATHWANI RA et al.** *Hepatology,* 2005, vol. 41, 380-382
- **KORONES DN et al.** *Am. J. Hematol.,* 2001, vol. 66, 46-48
- **BRUIX J et al.** *J Hepatol,* 2001, vol. 35, 421-430
- **DANIELE B et al.** *Gastroenterology,* 2004, vol. 127, 108-12
- **GEBO KA et al.** *Hepatology,* 2002, vol. 36, 84-92
- **KUTAY H et al.** *Cell Biochem,* 2006, vol. 99, 671-678
- **GRAMANTIERI L et al.** *Cancer Research,* 2007, vol. 67, 6092-6099
- **MENG F et al.** *Gastroenterology,* 2007, vol. 133, 647-658
- **VARNHOLT H et al.** *Hepatology,* 2008, vol. 47, 1223-1232
- **URA S et al.** *Hepatology,* 2009, vol. 49, 1098-1112
- **CHEN X-M.** *World J Gastroenterol,* 2009, vol. 15, 1665-1672
- **LEWIS AP ; JOPLING CL.** *Biochem. Soc. Trans,* 2010, vol. 38, 1553-1557
- **VANDESOMPELE J.** *Genome Biol,* 2002, vol. 3
- **ANDERSEN CL et al.** *Cancer Research,* 2004, vol. 64, 5245-5250
- **THÉRY C et al.** Curr Protoc Cell Biol. 2006
- **MESTDAGH P et al.** *Genome Biol,* 2009, vol. 10
- **VANDESOMPELE J ; KUBISTA M ; PFAFFL MW.** *Reference gene validation software for improved normalization,* 2008, 1-18
- **RANDALL G et al.** *Proc Natl Acad Sci USA,* 2007, vol. 104, 12884-12889
- **ARROYO JD et al.** *Proc Natl Acad Sci USA,* 2011, vol. 108, 5003-5008
- **CHANG J et al.** *RNA Biol,* 2004, vol. 1, 106-113
- **BERESFORD PJ.** *Journal of Biological Chemistry,* 2001, vol. 276, 43285-43293
- **CHOWDHURY D ; LIEBERMAN J.** *Annu Rev Immunol,* 2008, vol. 26, 389-420
- **KRÜTZFELDT J et al.** *Nature,* 2005, vol. 438, 685-689
- **WANG K et al.** *Proc Natl Acad Sci USA,* 2009, vol. 106, 4402-4407
- **ZHANG Y et al.** *Clinical Chemistry,* 2010, vol. 10, 1373
- **STARKEY LEWIS PJ et al.** *Hepatology,* 2011, vol. 54, 1767-1776
- **CERMELLI S et al.** *PLoS ONE,* 2011, vol. 6, 2393710-1371
- **GUI J et al.** *Clin. Sci.,* 2011, vol. 120, 183-193

- **KROH EM et al.** *Methods,* 2010, vol. 50, 298-301
- **RACANELLI V et al.** *Hepatology,* 2006, vol. 43, S54-S62
- **ABRAHAM C ; CHO JH.** *N Engl J Med,* 2009, vol. 361, 2066-2078
- **VALADI H et al.** *Nat Cell Biol,* 2007, vol. 9, 654-659
- **GIBBINGS DJ et al.** *Nature,* 2009, vol. 11, 1143-1149
- **GIBBINGS D et al.** *Trends Cell Biol,* 2010, vol. 20, 491-501
- **FORNARI F et al.** *Cancer Research,* 2009, vol. 69, 5761-5767
- **COULOUARN C et al.** *Oncogene,* 2009, vol. 28, 3526-3536
- **AFANASYEVA EA et al.** *Cell Death and Differentiation,* 2011, vol. 18, 974-984
- **LIU Z et al.** *Molecular Cell,* 2008, vol. 29, 665-678
- **ZENG R et al.** *J Proteome Res,* 2011